# EUROPEAN PATENT SPECIFICATION

(11) **EP 0 593 450 B1**
(45) Date of publication and mention of the grant of the patent: **17.07.1996**
(21) Application number: 91901056.1
(22) Date of filing: 21.11.1990
(51) Int. Cl.: A61K 38/00, C07K 14/00

(54) **COMPOSITIONS comprising omega conotoxin peptide derivatives and their use FOR TREATING ISCHEMIA-RELATED NEURONAL DAMAGE**
ZUSAMMENSETZUNGEN enthaltend omega Conotoxin Peptide Derivate und deren Verwendung ZUR BEHANDLUNG VON ISCHÄMIE-ARTIGEN NEURONALEN SCHÄDIGUNGEN
COMPOSITIONS comprenant des dérivés des oméga peptides de la conotoxine et leur utilisation dans lE TRAITEMENT DE DOMMAGES DES NEURONES DUS A L'ISCHEMIE

(30) Priority: 22.11.1989 US 440094
(43) Date of publication of application: 27.04.1994
(73) Proprietor: NEUREX CORPORATION, Menlo Park, CA 94025-1057 (US)
(72) Inventor: MILJANICH, George, P., Redwood City, CA 94062 (US); BITNER, Robert, S., West Lafayette, IN 47906 (US); BOWERSOX, Stephen, S., Menlo Park, CA 94025 (US); FOX, James, A., Palo Alto, CA 94306 (US); VALENTINO, Karen, L., San Carlos, CA 94070 (US); YAMASHIRO, Donald, H., Cleveland Heights, OH 44118 (US)
(74) Representative: Goldin, Douglas Michael
(86) International application number: PCT/US90/06853
(87) International publication number: WO 91/07980

(56) References cited:
- Science, vol. 244, 9 June 1989; G.T. Jones et al.: "Localization and mobility of omega-conotoxin-sensitive Ca2+ channels in hippocampal CA1 neurons", pages 1189-1193
- Biochemistry, vol. 26, 1987, American Chemical Society; B.M. Olivera et al.: "Neuronal calcium channel antagonissts. Discrimination between calcium channel subtypes using omegaconotoxin frm conus magus venom", pages 2086-2090
- Biochemistry, vol. 26, 1987, American Chemical Society; L.J. Cruz et al.: "Characterization of the omega-contoxin traget. Evidence for tissue-specific heterogeneity in calcium channel types", pages 820-824

## Description

### 1. Field of the Invention

The present invention relates to a composition for use in reducing neuronal damage associated with an ischemic condition, such as stroke, and to novel peptides contained in the composition.

### 2. References

Ahmad, S. et al., Brain Research, 453:247-256 (1988).

Aitkenhead, A. (1986), British Journal of Hospital Medicine, :290-296.

Baethmann and Jansen (1986), European Neurology 25, Supplement 1:102-114.

Bennett, J.P., et al, Neurotransmitter Receptor Binding, pp. 61-89, Raven Press, NY (1983).

Berger, L. and Hakim, A. (1988) Stroke, 19:1257-1261.

Brandt, L., Ljunggrenm B., Saveland, H., and Hyman, T. (1988), Acta Neurochirurgica, Supplement 45:11-20.

Brint, S., et al., J. Cerebral Blood Flow Metab. 8:474-485 (1988).

Gelmers, H., Gorter, K., Weerdt, C., Weizer, H. (1988), New England Journal of Medicine 318:203-207.

Goldberg, M., Weiss, J., Pham, P., and Choi, D. (1987), The Journal of Pharmacology and Experimental Therapeutics 243:784-791.

Gray, W., Olivera, B., and Cruz, L. (1988), Annual Review of Biochemistry 57:665-700.

Hartley, D. and Choi, D. (1989), The Journal of Pharmacology and Experimental Therapeutics 250:752-758.

Kirino, T., (1982) Brain Research, 239:57-69.

Langley, M. and Sorkin, E. (1989), Drugs 37:669-699.

McCleskey, E.W. et al., Proc. Natl. Acad. Sci. USA 84:4327-31 (1987).

Newberg, L., Steen, P., Milde, J., and Michenfelder, J. (1984), Stroke 15:666 et seq.

Nowycky, M.C., Fox, A.P., and Tsien, R.W., Nature (London), 316:440-443 (1985).

Pulsinelli, W.A., et al, (1979), Stroke, 10:267-272.

Olivera, B., Mcintosh, J., Cruz, L., Luque, F., and Gray, W. (1984), Biochemistry 23:5087-5090.

Olney, J., Labruyere, J., and Price, M. (1989), Science 244:1360-1362.

Rivier, J., et al., J. Biol. Chem. 262:1194-1198.

Rothman, S. (1984), Journal of Neuroscience 7:1884-1891.

Sano, K., et al (1987), Eur J Pharmacol, 141:235-241.

Simon, R., Swan, J., Griffiths, T., Meldrum, B. (1984), Science 226:850-852.

Tateishi, A., Fleischer, J., Drumond, J., Scheller, M., Zornow, M., Grafe, M., and Schapiro, H. (1989), Stroke 20:1044-1050.

Van Reempts, J. and Borgers, M. (1984), Acad. Anaesthesiolica Belge 35, Supplement, 209-218.

Van Reempts, J. and Borgers, M. (1985), Annals of Emergency Medicine 14:8-et seq.

Vibulsreth, S., Dietrich, W., Bustro, R., and Ginsberg, M. (1987), Stroke 18:210-216.

Wauquier, A., Edmonds, H., Clincke, G. (1987), Neuroscience and Biobehavioral Reviews 11:287-306.

### 3. Background of the Invention

Ischemic damage to the central nervous system (CNS) may result from either global or focal ischemic conditions. Global ischemia occurs under conditions in which blood flow to the entire brain ceases for a period of time, such as may result from cardiac arrest. Focal ischemia occurs under conditions in which a portion of the brain is deprived of its normal blood supply, such as may result from thromboembolytic occlusion of a cerebral vessel, traumatic head injury, edema, and brain tumors.

Both global and focal ischemic conditions have the potential for producing widespread neuronal damage, even if the ischemic condition is transient. Although some permanent neuronal injury may occur in the initial minutes following cessation of blood flow to the brain, most of the damage in global and focal ischemia occurs over hours or even days following the ischemic onset. Much of this neuronal damage is attributed to secondary consequences of reperfusion of the tissue, such as the release of vasoactive products by damaged endothelium, and the release of cytotoxic products (free radicals, leukotrienes, etc.) by damaged tissues.

Several drug strategies have been proposed for treatment of stroke and other neuronal conditions related to ischemia, and these have been reviewed in recent articles (e.g., Wauquier). Anti-coagulants, such as heparin, have been examined, but with mixed results. Similarly, antivasoconstriction agents such as flunarazine, excitatory neurotransmitter antagonists, such as MK-801 and AP7, and anti-edemic compounds have shown mixed results, with no clear benefits to outweigh a variety of side effects, including neurotoxicity psychosis or increased susceptibility to infection.

Two general classes of vasodilators have been studied for possible treatment of neuronal ischemic damage. Non-specific vasodilators, including papaverine, prostacyclin, pentoxifylline, and nitroprusside failed to demonstrate any clear benefit in reducing ischemic damage. A second general class of vasodilators includes a variety of calcium-antagonist vasodilator drugs. Verapamil and related compounds which prevent calcium entry into smooth and striated muscles appear to be effective only at high drug concentrations, where serious cardiotoxicity may ensue. Dihydropyridines, such as nimodipine, produced mixed results - some neurological improvement may be seen, but increased cerebral edema has also been observed. Benzothiazepines, as exemplified by diltiazem, have shown moderate protective effects, but these drugs also appear to cause undesired side efffects such as hypotension which may be inimical to treatment.

In summary, drugs which have been proposed to date for the treatment of stroke and other ischemic-related conditions of the brain are either (i) relatively ineffective, (ii) effective only at dosage levels where undesired side effects are observed, and/or (iii) produce systemic effects, such as hypotension, which compromise the potential effectiveness of the drug.

Biochemical studies have been carried out on omega conotoxin compounds and have been discussed by Jones et al (Science, 244, 1189-1193, 1989), Olivera et al (Biochemistry, 26, 2086-2090, 1987) and Cruz et al (Biochemistry, 26, 820-824, 1987).

Jones et al described the localization of voltage-dependent calcium channels, as defined by binding of omega-conotoxin derivatives containing fluorescent or biotin tags. Their studies addressed the localization and mobility of such binding sites.

Olivera et al described the purification, sequencing and synthesis of omega conotoxins MVIIA and MVIIB, as well as the binding characteristics of these compounds in chick, calf and frog brain, as compared to binding of omega conotoxin GVIA (CgTx). Omega conotoxin binding site heterogeneity in amphibian, but not mammalian brain, is described in this reference.

Cruz et al similarly described biochemical studies on omega conotoxin binding sites in brain tissue. The binding studies reported by Cruz differentiate the omega conotoxin binding site in brain from muscle dihydropyridine binding sites.

With reference to DE-A-39 26 287 published on 21.02.91 the applicant has voluntarily limited the scope of the present application in respect of claims for Germany.

### 4. Summary of the Invention

It is therefore a general object of the invention to provide a composition effective for treating neuronal damage related to ischemic injury in the brain.

Another object of the invention is to provide novel peptides for treating such neuronal injury.

The present invention comprises the use of a neuronal-cell calcium channel antagonist omega-conotoxin peptide having activities for blocking norepinephrine release in mammalian central nervous system (CNS) neuronal cells, and for binding to neuronal-membrane omega-conotoxin (OCT) MVIIA binding sites which are within the range of such activities for OCT's MVIIA, GVIA, and TVIA for the manufacture of a medicament for reducing neuronal damage related to an ischemic condition in a mammalian species. The peptide is carried in a vehicle suitable for injection.

One preferred OCT peptide has the form:
CKGKGAX₁CX₂RX₃X₄YDCCTGSCX₅RX₆GKC-t,
where X₁=K or S; X₂= S or H; X₃=L or T; X₄=M or S; X₅=N or a deletion; X₆=S or deletion; and t is a carboxy or amidated carboxy terminal group.

Another preferred OCT peptide has the form:
CX₁SXGSSCSXTSYNCCRSCNXYX₂X₃X₄CX₅-t,
where X₁=K or L; X₂=T or S; X₃=K or R; X₄=R or K; X₅=Y or R; and t= a carboxy or amidated carboxy terminal group.

More generally, the medicament includes a neuronal-cell calcium channel antagonist compound having activities for inhibiting norepinephrine release from mammalian CNS neuronal cells, and for binding to neuronal-membrane omega-conotoxin MVIIA binding sites with activities which are within the range of such activities for OCT's MVIIA, GVIA and TVIA. The compound is carried in a suitable pharmaceutical vehicle.

In one embodiment, the binding activity of the compound to the MVIIA site is characterized by a binding constant determined by competitive displacement of OCT MVIIA from neuronal membranes by the compound. In a second embodiment, the binding activity is characterized by a ratio of binding constants for compound binding to a neuronal-cell OCT SVIB site and a neuronal-cell OCT MVIIA site.

The invention further includes an OCT peptide having the form:
CKGKGAX₁CX₂RX₃X₄YDCCTGSCX₅RX₆GKC-t,
where X₁=K or S; X₂=S or H; X₃=L or T; X₄=M or S; X₅=N or a deletion; X₆=S or deletion; and t= a carboxy or amidated carboxy terminal group, excluding the peptides in which X₁=K, X₂=S, X₃=L, X₄=M, X₅=deletion, and X₆=S; and X₁=S, X₂=H, X₃=T, X₄=S, X₅=N, and X₆=deletion, or the form:
CX₁SXGSSCSXTSYNCCRSCNXYX₂X₃X₄CX₅-t,
where X₁=K or L; X₂=T or S; X₃=K or R; X₄=R or K; X₅=Y or R; and t= a carboxy or amidated carboxy terminal group, excluding the peptides in which X₁=K, X₂=T, X₃=K, X₄=R, and X₅=Y; and X₁=L, X₂=S, X₃=R, X₄=K, and X₅=R.

In still another aspect, the invention includes a method of selecting a calcium channel peptide antagonist compound effective in reducing the ischemic-related neuronal damage, such as produced by stroke, in a mammalian species. The compound is assayed for its affinity to the neuronal cell OCT MVIIA binding site, and for its ability to inhibit norepinephrine release in mammalian CNS neuronal cells. The assayed compound is selected for use in such treatment if its activities for inhibiting norepinephrine release from CNS neuronal cells, and for binding to neuronal-membrane omega-conotoxin MVIIA binding site are within the range of such activities for OCT's MVIIA, GVIA, and TVIA.

These and other objects and features of the invention will become more fully apparent when the following detailed description of the invention is read in conjunction with the accompanying drawings.

### Brief Description of the Drawings

Figure 1 shows primary sequences of several naturally-occurring OCT peptides;
Figure 2 shows analog OCT peptides;
Figure 3 shows voltage-gated calcium current traces induced by voltage steps from -100 or -80 mV to -20 mV in untreated neuroblastoma cells (3A) and in neuroblastoma cells exposed to increasing concentrations of OCT MVIIA (3B-3D);
Figure 4 plots the percent inhibition of peak inward calcium currents in neuroblastoma cells as a function of OCT MVIIA (circles) and OCT GVIA (triangles);
Figure 5 shows the inhibition of norepinephrine release from neuronal cells by potassium stimulation (solid bars) or in basal conditions (open bars) as a function of OCT MVIIA activity;
Figure 6 (A and B) is binding curves showing the amount of OCT MVIIA bound to rat synaptosomal membranes, as a function of OCT MVIIA concentration (6A), and the same data plotted as a Scatchard plot (6B);
Figure 7 shows computer-fit competitive binding curves for OCT peptide binding to the OCT MVIIA binding site in rat brain synaptosomes (bound [¹²⁵I] MVIIA as a percentage of bound [¹²⁵I] at zero nonradiolabel ligand is shown);
Figure 8 shows an SDS-PAGE autoradiogram of an * of rat synaptosomal membranes having covalently bound radiolabeled OCT MVIIA (lanes a and b) or covalently bound OCT GVIA (lanes c and d) added to the membranes in the presence (lanes b and d) or absence (lanes a and c of non-radiolabeled OCT;
Figure 9 shows computer-fit competitive binding curves for OCT binding to the OCT SVIB binding site in rat brain synaptosomes;
Figure 10A-10B are low-power micrographs of gerbil hippocampus CA1 region in animals after ischemia, and infusion of OCT MVIIA (10A) or drug vehicle (10B);
Figures 11A-11D are higher power micrographs of cells in the drug-treated ischemic animals (11A, 11C, 11D), in animals receiving vehicle alone (11B), in animals showing complete protection by OCT against ischemic cell damage (11C); and in animals showing partial protection by OCT against ischemic cell damage (11D);
Figure 12 shows the degree of neuroprotection in hippocampal CA1 cells observed with a variety of OCT peptides in a global ischemia model of neuroprotection;
Figure 13 plots the changes in spontaneous motor activity in animals which were (a) unoccluded and untreated (open circles), (b) unoccluded and treated with MVIIA peptide (closed circles), (c) occluded but untreated (open triangle) and (d) occluded and treated with MVIIA peptide (closed triangles); and
Figure 14 shows amino acid sequences of neuroprotective and inactive groups of OCT peptides.

### Detailed Description of the Invention

### I. OCT Peptides

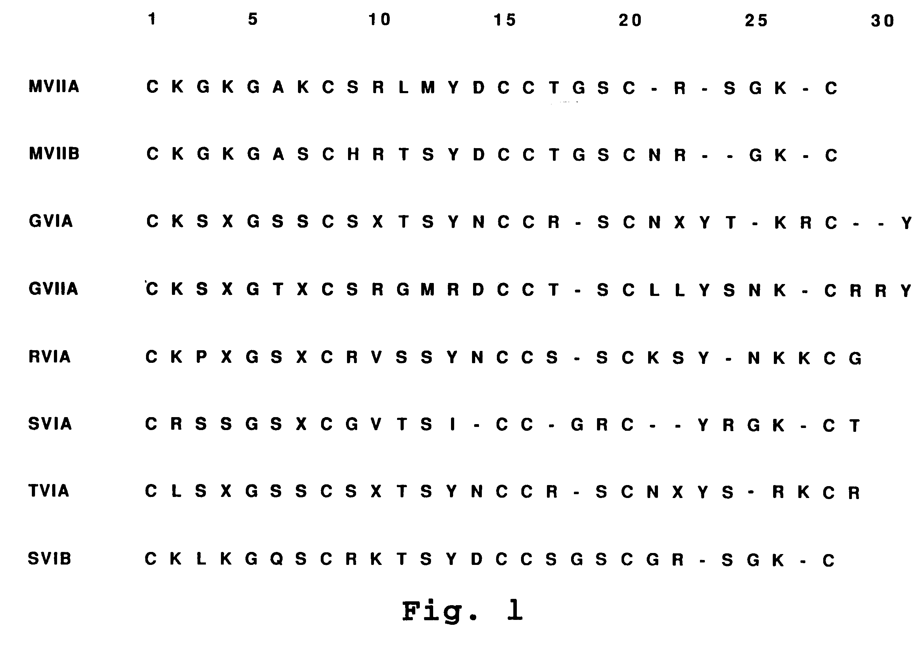

Omega-conotoxin (OCT) peptides are peptide toxins produced by marine snails of the genus Conus, and which act as calcium channel blockers (Gray). About 500 species of cone snails in the Conus genus have been identified, and a variety of OCT peptides from several of these species have been isolated. The primary sequences of seven natural OCT peptides are shown in Figure 1. Conventional letter initials are used for the amino acid residues, and X represents 4-hydroxyproline, also abbreviated 4Hyp. All of the peptides shown in the figure are amidated at their C-termini.

The identifying names of the peptides are also given in the figure, and these names will be used herein to refer to the specific OCT peptide. For example, the peptide whose sequence is designated MVIIA will be referred to herein as OCT MVIIA. The OCT MVIIA and OCT GVIA peptides also have the common names CmTx and CgTx, respectively. All of the OCT peptides have three disulfide linkages connecting cysteine residues 1 and 4, 2 and 5, and 3 and 6, as indicated for the MVIIA peptide.

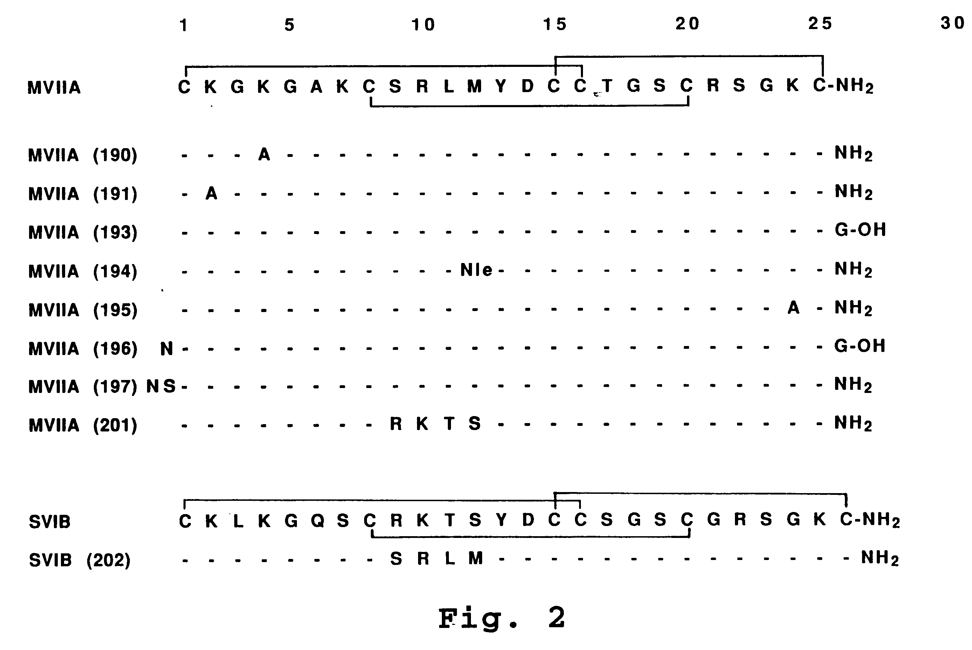

Figure 2 shows analogs of natural OCT MVIIA, OCT GVIA, and SVIB peptides which have been synthesized and tested in accordance with the invention. Standard single amino acid code letters are used in the figure; X=hydroxyproline; Nle=norleucine; NH₂ group at the C terminus indicates that the peptide is C-terminal amidated; G-OH indicates termination in an unmodified glycine residue.

OCT peptides, such as those shown in Figures 1 and 2, can be synthesized by a conventional solid phase methods, such as have been described (Rivier). Briefly, N-alpha-protected amino acid anhydrides are prepared in crystallized form and used for successive amino acid addition at the N-terminus. At each residue addition, the growing peptide (on a solid support) is acid treated to remove the N-alpha-protective group, washed several times to remove residual acid and to promote accessibility of the peptide terminus to the reaction medium. The peptide is then reacted with an activated N-protected amino acid symmetrical anhydride, and the solid support is washed. At each residue-addition step, the amino acid addition reaction may be repeated for a total of two or three separate addition reactions, to increase the percent of growing peptide molecules which are reacted. Typically, 1-2 reaction cycles are used for the first twelve residue additions, and 2-3 reaction cycles for the remaining residues.

After completing the growing peptide chains, the protected peptide resin is treated with liquid hydrofluoric acid to deblock and release the peptides from the support. For preparing an amidated peptide, the resin support used in the synthesis is selected to supply a C-terminal amine, after peptide cleavage from the ring.

The three disulfide linkages in the peptides may be formed by air oxidation in the presence of dithiothreitol (DTT) at room temperature or at 4°C over an extended reaction period. Alternatively, where the correct or desired bridging cannot be achieved by random oxidation, a chemically directed process may be used in which the bridges are formed sequentially, one bridge at a time. The following side-chain protecting groups could be used for each pair of cysteine residues: 4-methylbenzyl, ethylcarbamoyl, and acetamidomethyl. These protecting groups constitute an orthogonal set in which any one kind of protecting group can be removed under conditions that do not affect the other two. The strategy here involves removing one kind of protecting group from a pair of cysteine residues, followed by oxidation to form the first disulfide bridge. A second kind of protecting group is then removed, again followed by oxidation to form the second bridge. A third bridge is formed in like manner.

The peptide can be isolated by an initial separation by gel filtration, to remove peptide dimers and higher polymers, and also to remove undesired salts, such as guanidine hydrochloride, used in the oxidation reaction. The partially purified peptide is further purified by preparative HPLC chromatography, and the purity of the peptide confirmed by amino acid composition analysis.

### II. Neuroprotective Compounds

This section describes in vitro inhibitory and binding properties of compounds, as exemplified by OCT peptides, which are effective in reducing ischemia-related neuronal damage, in accordance with the invention. As will be discussed in Section V below, the in vitro inhibitory and binding properties can be used in selecting neuroprotective compounds.

### A. Calcium-Channel Antagonist Activity

The neuroprotective compounds of the invention are neuronal-cell calcium channel antagonists, as defined by their ability to inhibit voltage-gated ionic currents in neuronal cells.

Voltage-gated calcium channels are present in neurons, and in cardiac, smooth, and skeletal muscle and other excitable cells, and are known to play a variety of roles in membrane excitability, muscle contraction, and cell secretion, such as in synaptic transmission (McCleskey). In neuronal cells, voltage-gated calcium channels have been classified into L, T, and N channels, each with characteristic gating voltage, inactivation rate, and selective modulation by neurochemicals (Nowycky).

One suitable system for testing inhibition (blockage) of voltage-gated neuronal calcium channels is the mouse neuroblastoma cell line, strain N1E115. Membrane currents are conveniently measured with the whole cell configuration of the patch clamp method, according to the procedure detailed in Example 1. Briefly, a voltage clamp protocol was performed in which the cell potential was stepped from the holding potential of about -100 mV to test potentials that ranged from -60 mV to +20 mV, and the cell was held at the holding potential for 5 seconds between pulses.

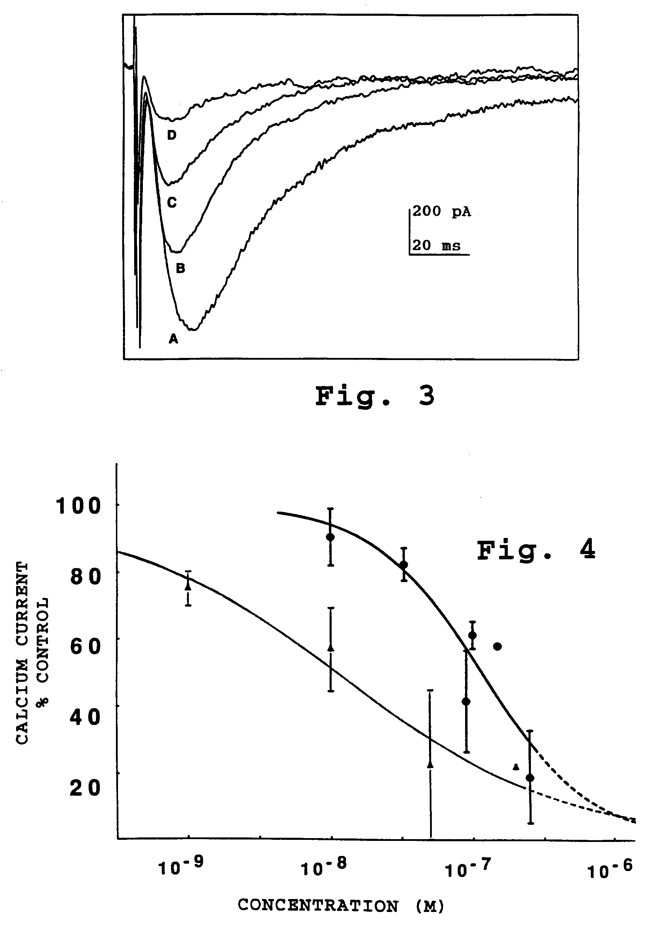

Figure 3 shows a typical inward calcium current elicited by a voltage step from -80 mV to -20 mV in the absence of OCT. In this, and most of the recordings, Ba replaced Ca as the charge-carrier through the calcium channels in order to increase the signal (McCleskey). According to the procedure described in Example 1, an N1E115 neuroblastoma cell wasbathed in saline with sodium replaced by N-methyl-D-glucamine (NMDG), and 10 mM Ba instead of 2 mM Ca. These substitutions reduced the sodium current that would otherwise have contaminated the calcium current record, and increased the calcium current above what it would have been with only 2 mM Ca in the bath. Potassium currents were blocked by TEA in the bath and Cs in the pipet solution.

As seen from Figure 3, curve A, the calcium current activates quickly (within about 20 ms) and inactivates with a time constant of 30 to 40 ms. The calcium current is measured by the amplitude of the peak inward current elicited by the depolarization peak, and has a measured value of about -1200 pA. The cell in Figure 3 (curve A) was also exposed to 1µM nifedipine, a dihydropyridine, which is expected to effectively block L-type calcium channels in the neuroblastoma cells, and no effect on the measured calcium current was observed. The calcium current observed is thus expected to be predominantly an N-type calcium channel current.

The responses of voltage-gated calcium current to increasing dosages of OCTs MVIIA and GVIA are shown in Figure 4. The ED₅₀ concentration, at which 50% inhibition of calcium current is produced, is determined from the voltage-gated current amplitudes, plotted as a function of OCT peptide concentration. The calculated ED₅₀ is 12 nM for GVIA and 116 nM for MVIIA, indicative of high inhibitory peptide activity. The ED₅₀ concentration for these and OCT peptides SVIA and SVIB are given in Table 1 below. The two compounds with relatively low IC₅₀ values (below 1 µM) are both active as neuroprotective agents, as will be seen in Section III below, whereas the OCT SVIA and SVIB peptides with IC₅₀ values above this threshhold are not. More generally, the compounds of the invention are classified as antagonists of voltage-gated calcium channels by their ability to inhibit voltage-gated calcium channel currents with an ED₅₀ value of less than about 1 µM in the assay detailed in Example 1.

**Table 1**

| Inhibition of calcium currents in N1E-115 neuroblastoma cells | |
|---|---|
| Compound | IC₅₀ |
| OCT GVIA | 12nM |
| OCT MVIIA | 116nM |
| OCT SVIB | > 1µM |
| OCT SVIA | >20µM |

Test peptides which are inhibitory for neuronal cell calcium currents can be further tested in non-neuronal cells, to confirm that the peptide activity in blocking calcium currents is specific to neuronal cells. A variety of muscle cell types which are refractory to calcium-current inhibition by OCTs, such as vertebrate embryo heart and skeletal muscle cells, are suitable. Cell current measurements are made substantially as outlined above and detailed in Example 1. OCT MVIIA, for example, has been reported to block voltage-gated calcium channels in a variety of neuronal cells, including dorsal root ganglion (DRG) neurons (McCleskey). This blockage or inhibition of calcium channel currents has been reported to be neuron-specific, since calcium current inhbition by the peptide was not observed in cardiac, smooth, and skeletal muscles.

### B. Selective Inhibition of Norepinephrine Release

A second requisite property of neuroprotective compounds, in accordance with the invention, is the ability to specifically inhibit depolarization-evoked and calcium-dependent norepinephrine release in brain (CNS) neuronal cells, but not inhibit neurotransmitter relerase at a mammalian neuromuscular junction of a skeletal muscle. Inhibition of norepinephrine release in neuronal cells can be assayed in mammalian brain hippocampal slices by standard methods, such as detailed in Example 2. Briefly, hippocampal slices are distributed to individual wells of a microtitre plate, and incubated with radiolabeled norepinephrine under conditions favoring cell uptake. The cells are washed with a low-potassium medium, then bathed for 15 minutes in a stimulation medium, in the presence of selected concentrations of the test compound. After removal of the stimulation buffer, radioactivity remaining in each slice is determined.

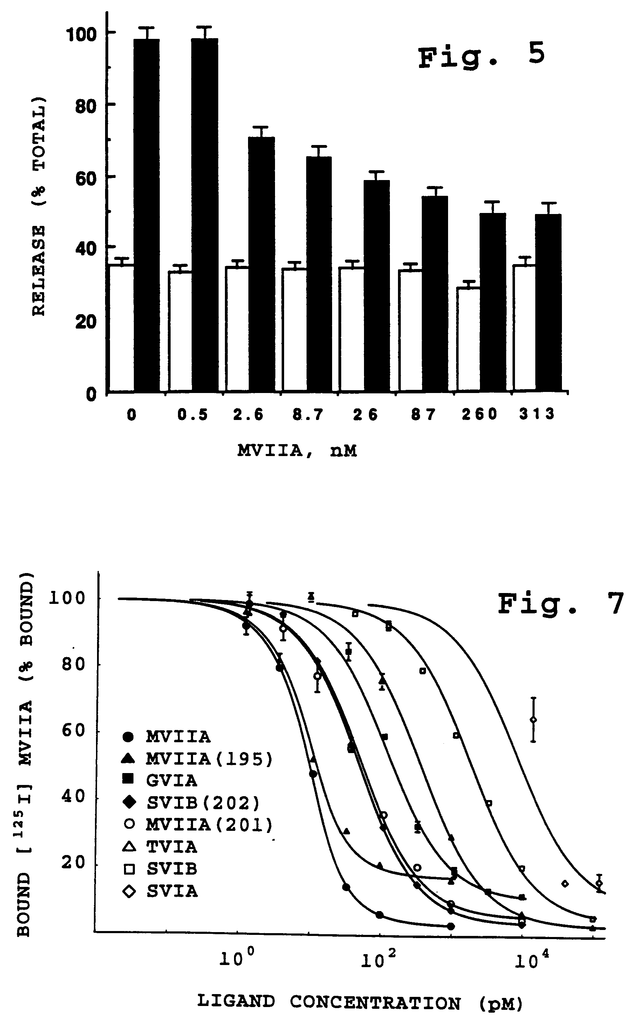

Figure 5 shows effects of increasing concentrations of OCT MVIIA peptide on norepinephrine release from rat brain hippocampal slices which were first bathed in normal wash solution (open bars), then stimulation medium (solid bars). As seen, the compound produces a strong dose-dependent inhibition of norepinephrine release in the presence, but not in the absence of stimulation medium. From the dose-dependent inhibition data, the compound concentration effective to produce 50% inhibition of epinephrine release is calculated.

The IC₅₀ values given in Table 2 for a variety of OCT peptides which have been examined by this method represent the average IC₅₀ values calculated from thin (200 µ) and thick (400 µ) hippocampal slices. The three lowest IC₅₀ values, between 0.8 and 2.4 nM, correspond to OCT peptides which show pronounced neuroprotective activity (Section III below). The OCT peptides MVIIA(195) and MVIIA(201) are MVIIA with amino acid substitutions or modifications at key residue sites (Figure 2), as will be discussed in Section IV below. The higher IC₅₀ values measured for these modified peptides is reflected in substantial reduction or loss of neuroprotective activity. The SVIA and SVIB OCT peptides are representative of OCT compounds which show no neuroprotective activity, and this is reflected by high IC₅₀ values for norepinephrine release. The SVIB(202) peptide is a modification of SVIB peptide in which the Ser-Arg-Leu-Met residues at positions 9-12 in MVIIA OCT are substituted for the Arg-Leu-Thr-Ser residues at the same positions in the SVIB OCT. This modification significantly reduced the IC₅₀ value for inhibition of norepinephrine release, and only weak neuroprotective activity was observed.

**Table 2**

| Inhibition of norepinephrine release by OCT Peptides | |
|---|---|
| OCT Peptides | IC₅₀ (nM) |
| SNX-195 (A²⁴MVIIA) | 0.17 |
| GVIA | .82 |
| MVIIA | 1.47 |
| TVIA | 2.4 |
| MVIIA(201) | 10.5 |
| MVIIA(195) | 11.0 |
| SVIB(202) | 29.0 |
| SVIB | 200.0 |
| MVIIA(191) | >100 |
| SVIA | >4500 |

In summary, pronounced neuroprotective activity is associated with an ability to inhibit norepinephrine release with IC₅₀ in the range 0.8-2.4nM, and more generally with IC₅₀ value which is within the range of IC₅₀ values measured for active OCT's MVIIA, GVIA, and TVIA; i.e., less than the largest of the IC₅₀ values measured for these active OCT peptides. Compounds with IC₅₀ values slightly outside this range may have moderate to low neuroprotective activity, and compounds with high IC₅₀ values are not neuroprotective.

### C. Specific, High Affinity Binding to OCT Receptors

Another property of neuroprotective compounds, in accordance with the invention, is high-affinity binding for an OCT MVIIA binding site in neuronal cells. As will be seen below, the binding affinity can be characterized either by the binding constant of the compound for the MVIIA binding site, or by the ratio of binding constants measured for binding to neuronal-cell MVIIA binding site and SVIB binding site.

Binding to OCT MVIIA binding site in neuronal tissue can be demonstrated with a variety of cell types and synaptosomal cell fractions. One preferred neuronal membrane is a mammalian brain synaptosomal preparation, such as the rat brain synaptosome preparation described in Example 3. The binding constant of a compound for the MVIIA binding site is typically determined by competitive displacement of radiolabeled OCT MVIIA from the synaptosomal preparation, as follows.

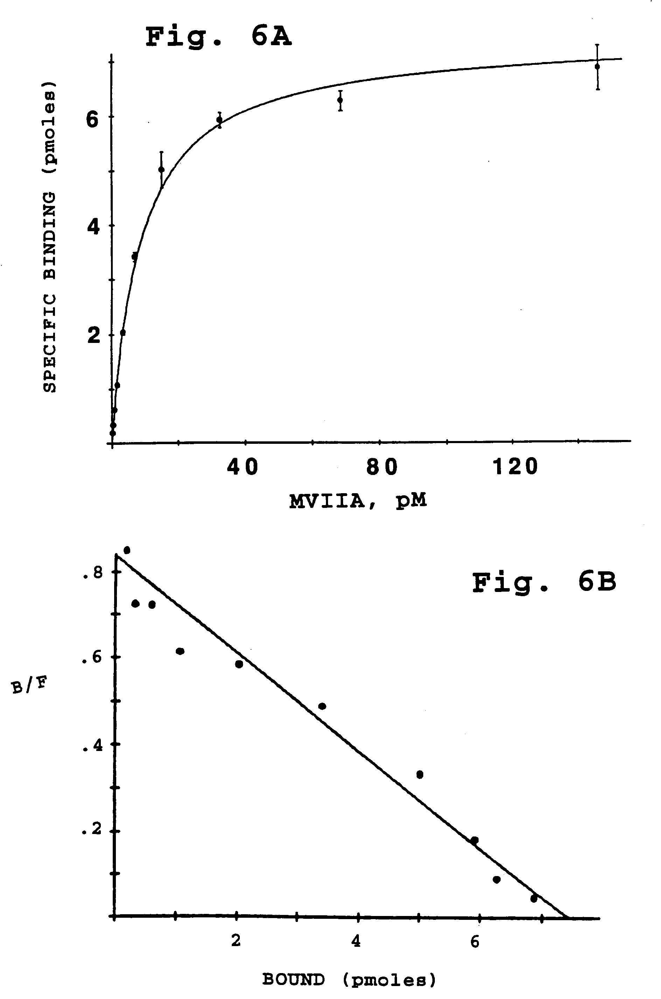

The binding constant K_{d} of the MVIIA peptide for the synaptosomal membranes is determined by a saturation binding method in which increasing quantities of radiolabeled peptide are added the synaptosomal membranes, and the amount of labeled material bound at each concentration is determined. The plot of bound peptide as a function of concentration is then used to calculate a Bₘₐₓ, the concentration of binding sites on the synaptosomes, and K_{d} following standard methods. In particular, the K_{d} value is the calculated concentration of peptide needed to half saturate the synaptosomal specific binding sites. Figure 6A shows the specific binding of OCT MVIIA to rat brain synaptosomes, plotted as a function of OCT peptide concentration, and Figure 6B, the same data in Scatchard plot form. From the slope of the Scatchard plot line, a K_{d} binding value of 8.8 pM is obtained.

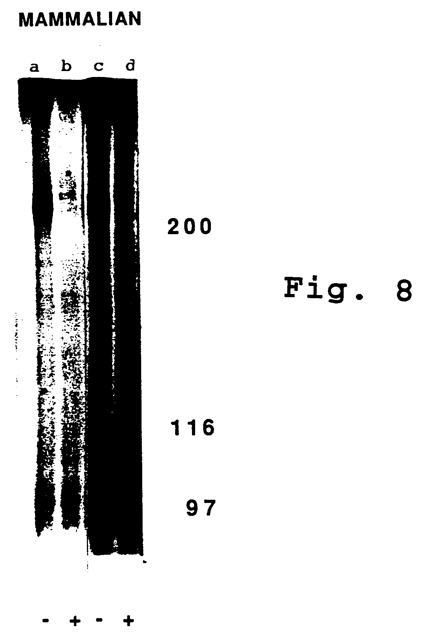

To determine the binding constant of a test compound for the MVIIA binding site, the test compound is added, at increasing concentrations in the nM range, to the synaptosome preparation having bound, radiolabeled OCT MVIIA. The synaptosomal material is then rapidly filtered, washed and assayed for bound radiolabel. The binding constant (Kᵢ) of the test compound is determined using computer-fit competitive binding curves, such as shown in Figure 7 for GVIA peptide, to determine first the IC₅₀ value of the compound, i.e., the concentration which gives 50% displacement of labeled MVIIA peptide, then calculating Kᵢ from the K_{d} value of OCT MVIIA and the IC₅₀ value of the compound, as detailed in Example 3. Calculated IC₅₀ and Kᵢ values for a number of OCT peptides which were examined are given in Table 3. The compounds are arranged in order of increasing IC₅₀ and Kᵢ values.

**Table 3**

| Competition of ¹²⁵I MVIIA binding by OCT peptides | | |
|---|---|---|
| | IC₅₀ (nM) | Ki(nM) |
| MVIIA(195) | .0092 | .0011 |
| MVIIA | .0145 | .0012 |
| MVIIA(194) | .0125 | .0022 |
| MVIIA(190) | .021 | .0056 |
| GVIA | .134 | .0093 |
| MVIIA(200) | .039 | .0115 |
| MVIIA(201) | .046 | .013 |
| SVIB(202) | .049 | .014 |
| MVIIA(193) | .070 | .020 |
| MVIIB | .103 | .036 |
| MVIIA(198) | .153 | .055 |
| MVIIA(191) | .165 | .065 |
| TVIA | .297 | .099 |
| MVIIA(196) | .426 | .151 |
| RVIA | .894 | .404 |
| SVIB | 1.74 | .549 |
| GVIIA | 3.69 | 1.34 |
| MVIIA(197) | 11.8 | 4.10 |
| SVIA | 1450. | 377. |

The MVIIA(198) compound contains an Ala substitution at position 2 in the OCT MVIIA, and the OCT MVIIA(200), an Ala substitution at the 7 position of OCT MVIIA. The other compounds are identified in Figure 2.

The compounds with known neuroprotective activity, OCT MVIIA, GVIA, and TVIA, have IC₅₀ values between about 15 and 300 pM, and Kᵢ values between about 1 and 100pM. Conversely, OCT peptides, such as OCT SVIA and SVIB, which are not neuroprotective have substantially greater IC₅₀ and Kᵢ values.

A number of OCT peptide compounds which were tested gave IC₅₀ and Kᵢ values within the ranges of those of OCT peptides MVIIA, GVIA, and/or TVIA, and these compounds should thus be considered candidates as neuroprotective compounds. However, some of these compounds, such as MVIIA(201), MVIIA(195), and SVIB(202) have IC₅₀ values for inhibition of norepinephrine release which are outside the range of neuroprotective compounds (Table 2), and thus these compounds do not meet all of the criteria for neuroprotective compounds.

The identity of the MVIIA binding protein in neuronal-cell membranes cells was examined by binding radiolabeled OCT MVIIA to synaptosomes, and crosslinking peptide to labeling neuronal membranes, as detailed in Example 5. The labeled membranes were solubilized with sodium dodecyl sulfate (SDS), fractionated by polyacrylamide gel electrophoresis (PAGE), and examined by autoradiography for labeled bands. In one case, the membranes were incubated with labeled peptide in the presence of excess unlabeled OCT MVIIA. A similar binding study was carried out with labeled OCT GVIA.

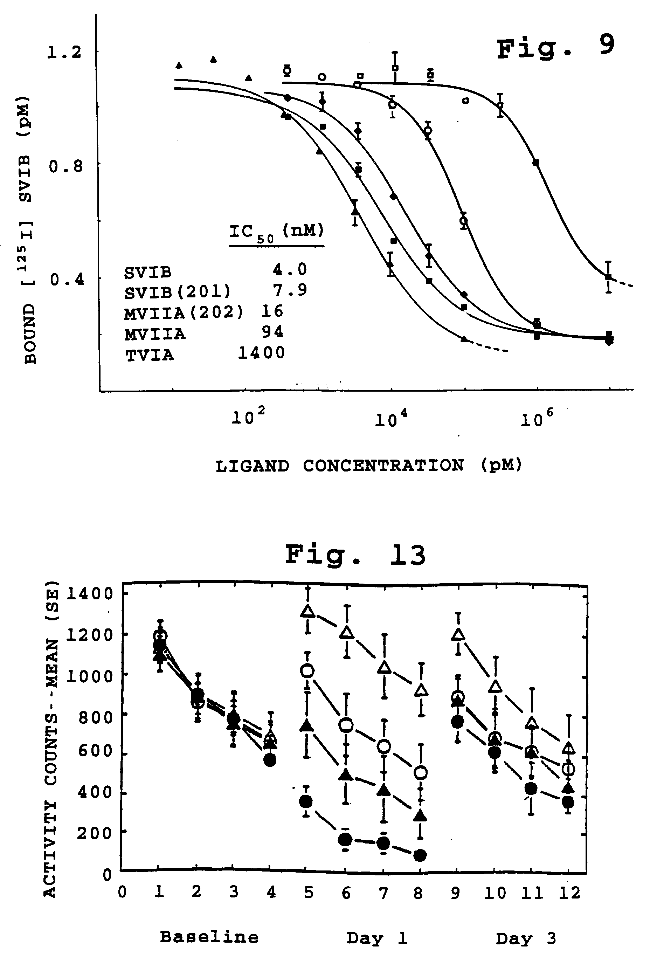

Autoradiographs of the gels are shown in Figure 8, where lanes a and b show MVIIA binding patterns to synaptosomal membranes in the absence (lane a) and presence (lane b) of unlabeled OCT MVIIA, and lanes c and d show GVIA binding patterns to synaptosomal membranes in the absence (lane c) and presence (lane d) of unlabeled OCT GVIA. The gel patterns show (lane a) most of the labeled MVIIA peptide binds to a 200-210 kilodalton protein band, and that the binding is specific, as evidence by displacement with unlabeled OCT MVIIA (lane b). This binding also appears to be a major specific binding site of OCT GVIA, as judged from the radiolabeled patterns in lanes c and d.

It has also been discovered, in accordance with the invention, that compounds with highest neuroprotective activity show relatively low binding affinity for an SVIB binding site on neuronal membranes, whereas high binding affinity for this site is observed with inactive compounds. IC₅₀ and Kᵢ values for compound binding to a neuronal cell SVIA binding site can be calculated, as above, by determining the K_{d} of OCT SVIB binding to a synaptosome preparation, then using competitive displacement of labeled OCT SVIB by the test compound, to determine the IC₅₀ and Kᵢ values of the test compound. Figure 9 shows computer-fit competitive binding curves for several OCT peptides whose binding to the SVIB binding site was examined. From these curves, IC₅₀ and Kᵢ values were determined as above.

Table 4 shows the Kᵢ values for the compounds examined. As seen, lowest affinity binding (highest binding constant values) was observed for neuroprotective compounds (OCT MVIIA, GVIA, and TVIA), and highest affinity, for non-protective compounds, e.g., SVIA and SVIB. Although a general inverse correlation between neuroprotective activity and SVIB binding is observed with some of the modified compounds, e.g., MVIIA(201), it is not seen with others, e.g., MVIIA(195). Table 4 also lists the Kᵢ of the OCT compounds for the MVIIA binding site, from Table 3, and the corresponding Kᵢ ratios Kᵢ(SVIB)/Kᵢ (MVIIA). These ratios accentuate the difference in binding properties between neuroprotective compounds, and those which show no neuroprotective activity.

**Table 4**

| Ratio of K₁ Values: | | | |
|---|---|---|---|
| Compound | Kᵢ(MVIIA) (nM) | Kᵢ(SVIB) (nM) | Kᵢ(MVIIA)/Kᵢ(SVIB) |
| SVIB | .549 | 4.03 | 1.4 x 10⁻¹ |
| SVIA | 377. | 5,000. | 7.5 x 10⁻² |
| MVIIA(201) | .013 | 9.63 | 1.3 x 10⁻³ |
| SVIB(202) | .014 | 14.33 | 9.8 x 10⁻⁴ |
| GVIA | .0093 | 140. | 6.6 x 10⁻⁵ |
| TVIA | .099 | 1630. | 6.1 x 10⁻⁵ |
| MVIIA | .0012 | 123. | 9.76 x 10⁻⁶ |
| MVIIA(195) | .0011 | 170. | 6.5 x 10⁻⁵ |

From the foregoing, it is seen that neuroprotective compounds in accordance with the invention are characterized by a high binding affinity for the MVIIA binding site on neuronal membranes. The binding affinity for this site may be characterized in one of two ways. In the first approach, the binding affinity of the compound for the MVIIA site is compared directly with that of OCT's MVIIA, GVIA, or TVIA. A neuroprotective compound is one of whose binding affinity is within the range of binding affinities measured for the OCT's MVIIA, GVIA, and TVIA, i.e., the binding constant is no greater than the highest binding constant among these three OCT peptides.

Alternatively, the binding affinity for the MVIIA site can be characterised by the ratio of binding constants for the MVIIA and SVIB sites, as just described. Here a neuro-protective compound is one whose binding ratio is within the range of such binding ratios measured for the OCT's MVIIA, GVIA, and TVIA, i.e., the binding ratio is no greater than the largest ratio among these three OCT peptides.

### III. Neuroprotective Compositions

The present invention provides a composition effective to reduce neuronal damage related to an ischemic condition in a human patient. The ischemic conditions may be due to an interruption in cerebral circulation, such as caused by cardiac failure, or other condition leading to global loss of blood supply to the brain, or to localized interruptions in blood flood, such as due to cerebral hemorrhaging, or localized thrombotic or embolic events, or head trauma.

The ischemic condition which is to be treated with the composition is generally associated with stroke, defined as the sudden diminution or loss of neurological function caused by an obstruction or rupture of blood vessels in the brain. In stroke, as well as in other types of cerebral ischemic conditions, the peptide treatment is aimed at preventing or reducing secondary brain damage resulting from the original ischemic event. The secondary damage typically includes cerebral cell destruction, or lesions, in the area surrounding the ischemic injury, in the case of focal ischemia, and also in areas of selective vulnerability in lesions, such as the hippocampus or basal ganglia, in the case of global ischemia. The secondary damage may often be manifested by functional impairment, such as loss of short-term or long-term memory. As will be seen below, the treatment method of the invention is effective in reducing or preventing both anatomical and functional secondary damage related to ischemia.

The composition of the invention includes a neuronal-cell calcium channel antagonist compound having activities for selectively blocking norepinephrine release in mammalian neuronal cells, and for binding to neuronal-membrane omega-conotoxin MVIIA binding site, which are within the ranges of such activities for OCT peptides MVIIA, GVIA, or TVIA. The binding activities may be expressed either as binding constants for the MVIIA site on neuronal membranes, or as a ratio of the binding constants for the MVIIA and SVIB binding sites, as discussed in Section II above. The compound is carried in a suitable pharmaceutical carrier, such as a sterile injectable solution.

One exemplary class of neuronal cell calcium channel antagonists is OCT peptides having the requisite inhibitory and binding activities. The peptide is formulated for parenteral administration in a suitable inert carrier, such as a sterile physiological saline solution. The concentration of peptide in the carrier solution is typically between about 0.1-10 mg/ml. The dose administered will be determined by route of administration. One suitable route is intracerebroventricular (ICV), at a dose level of about 0.1 to 50 µg peptide/kg body weight, depending on the binding and inhibitory values of the peptide. The peptide compound may alternatively be administered intravenously (IV) as demonstrated below. It may be desirable for IV administration to pretreat the subject with antihistamines specific for H1 and H2 histamine receptorss, to reduce possible blood pressure lowering after peptide administration.

As reported below, and according to an important feature of the invention, it has been found that there is little or no loss of protective effect of the neuroprotective compound when it is administered well after the ischemic event e.g., one hour following the period of transient occlusion. The delayed-administration protective event indicates that the peptide is effective in blocking the events leading from ischemic injury to secondary cerebral injury, since these events may occur over a period of many hours or even days after injury. Thus, the delayed administration may be effective to reduce secondary cerebral damage over a several hour period, or even a day or more, following the onset of ischemia.

The effectiveness of the composition in reducing neuronal damage related to ischemic injury has been examined in three animal systems which are widely employed as model systems for global ischemia and secondary stroke damage. The first system is the gerbil two vessel occlusion model of global ischemia produced by transient occlusion of carotid arteries of the neck. For clinical comparisons, the ischemia produced in this model has been likened to that produced by cardiac arrest, since all blood flow to the brain is stopped for a fixed period, typically 5-10 minutes.

Although some differences in particular sequelae have been noted among species, gerbils exhibit the same kind of selective regional damage to ischemia as is found in other mammals, including humans. In particular, the characteristic secondary damage observed in the hippocampal CA1 region is similar to that seen in other mammals, including humans (Kirino; Yamaguchi). Neurons in this area, and especially pyramidal neurons, exhibit a delayed neuronal death over a period of up to 4 days after ischemic injury.

The second model is the rat four-vessel occlusion model. The experimental procedure for producing temporary occlusion produces an ischemia that mimics conditions in the human brain following cardiac arrest, including the following similarities: the ischemic event is temporary, typically 5-30 minutes; it occurs in an unanesthetized state; in most rats, the ischemic event is not accompanied by generalized seizures, and animals that have seizures can be excluded from the study. In addition, the occlusion procedure allows the animals to be easily monitored, maintained and analysed (Pulsinelli).

The third model is the rat cerebral artery occlusion model of focal ischemia. In this model, the left cerebral artery is permanently occluded by electrocoagulation. Twenty four hours after the occlusion, the animals are anesthetized and areas of damage are examined by magnetic resonance imaging.

### A. Reduction in Anatomical Damage

Ischemia in the gerbil model system was induced in anesthetized animals by occluding the two carotid arteries for eight minutes, as detailed in Example 7. OCT peptide was administered ICV during the occlusion period, or one hour following occlusion. Four days after occlusion and peptide treatment, the animals were examined histologically for anatomical damage in the hippocampal CA1 region, as detailed in Example 7.

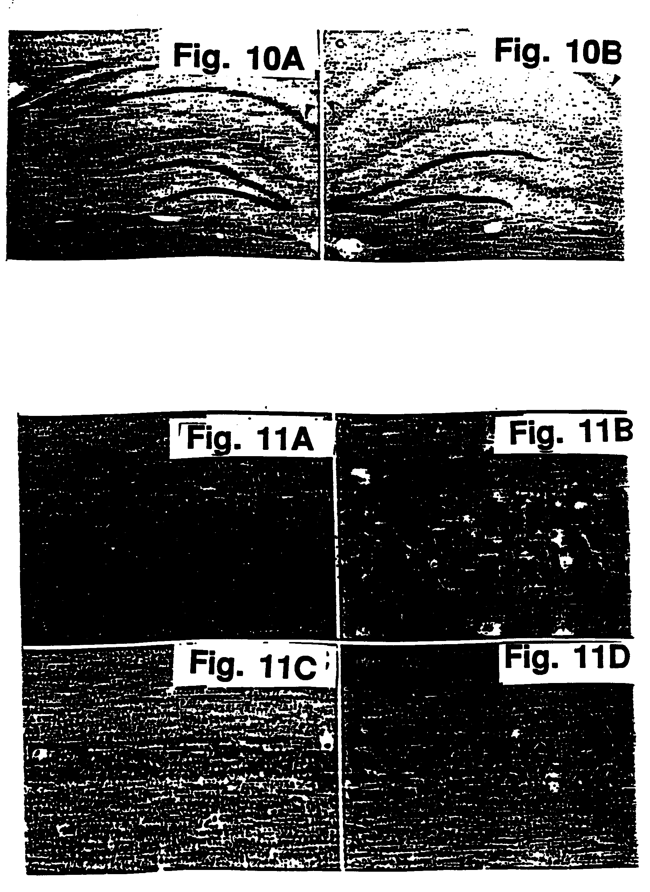

Figures 10A and 10B are low-power micrographs of gerbil hippocampus CA1 region in animals after ischemia, and infusion of MVIIA OCT (10A) or drug vehicle (10B). The arrows in the figures indicate the approximate borders of the CA1 region. At higher power, cells in the drug-treated ischemic animals appear normal (Figure 11A), whereas damage is apparent in the ischemic animals receiving vehicle alone (Figure 11B). Another example of complete drug protection is seen in Figure 11C, and an example of partial protection is seen in Figure 11D, where there are a small number of damaged cells.

Anatomical sections, such as those seen in Figures 10 and 11, were scored according to the criteria set out in Example 7. The extent of anatomical damage in ischemic animals treated with OCT MVIIA or OCT GVIA or receiving vehicle alone (control), based on the above scoring system, is given in Table 5 below. The peptide was administered by ICV infusion during the eight minutes of ischemia, at a total dose indicated in the table below. As seen, the extent of damage in the higher-dose OCT MVIIA treated animals was only 25% of that in untreated animals. The GVIA peptide also produced more than a 50% reduction in damage, and the lower dose was near maximal effectiveness.

**Table 5**

| Effect of OCT Peptides on Hippocampal Damage in Gerbils | | | |
|---|---|---|---|
| Treatment | N | (SEM) Mean Score | Percent Damage |
| Vehicle | 20 | 3.1 (.32) | 100% |
| 0.02 µg MVIIA | 4 | 1.9 (.83) | 61% |
| 0.1 µg MVIIA | 18 | 0.8 (.09)*** | 25% |
| 0.02 µg GVIA | 3 | 1.3 (.33)* | 42% |
| 0.1 µG GVIA | 11 | 1.2 (.39)** | 39% |

| | | | |
|---|---|---|---|
| * p< .05 compared to vehicle | | | |
| ** p< .005 compared to vehicle | | | |
| *** p< .0005 compared to vehicle | | | |

A similar treatment method was applied in the gerbil global ischemia model, but in which the neuroprotective agent was administered 1 hour after the ischemic event. The observed reduction in anatomical damage is summarized in Table 6 below. A comparison of the data in Table 5 indicates little loss of protective effect at a comparable dose (0.1 µg) when the drug is administered 1 hour after the ischemic event (8 min of occlusion).

**Table 6**

| Effect of OCT MVIIA on Hippocampal Damage in Gerbils (1 hour post-ischemia) | | | |
|---|---|---|---|
| Treatment | N | (SEM) Mean Score | Percent Damage |
| Vehicle | 15 | 3.0 (.31) | 100% |
| 0.1 ug MVIIA | 16 | 0.9 (.13)** | 30% |
| 0.3 ug MVIIA | 3 | 0.7 (.17)*** | 23% |

| | | | |
|---|---|---|---|
| ** p< .005 compared to vehicle | | | |
| *** p< .0005 compared to vehicle | | | |

Ischemia in the rat model system was induced by first surgically closing the vertebral arteries, and after surgical recovery, transiently blocking the carotid arteries (thus completely blocking blood flow to the brain) for a period of 15 minutes. During occlusion, animals were given 0.3 µg OCT MVIIA peptide ICV. Four days after occlusion, the animals were examined histologically to determine the extent of damage in the hippocampal CA1 region, as above. The mean scores are given in Table 7 for a comparison of saline and MVIA OCT treatments. As seen, the extent of damage in the treated animals was only about 1/3 that in untreated animals.

**Table 7**

| Effect of OCT MVIIA on Hippocampal Damage in Rats by 4-VO (15 min. 4-VO) | | |
|---|---|---|
| Treatment | N | Mean Score (SEM) |
| vehicle | 4 | 3.6 (0.38) |
| MVIIA OCT (0.3 µg) | 5^{a} | 1.2 (0.36)** |

| | | |
|---|---|---|
| ^{a} Animals given MVIIA ICV were included in the study only if they exhibited characteristic shaking behavior. | | |
| ** p<.005, unpaired Student's 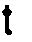 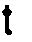 test. | | |

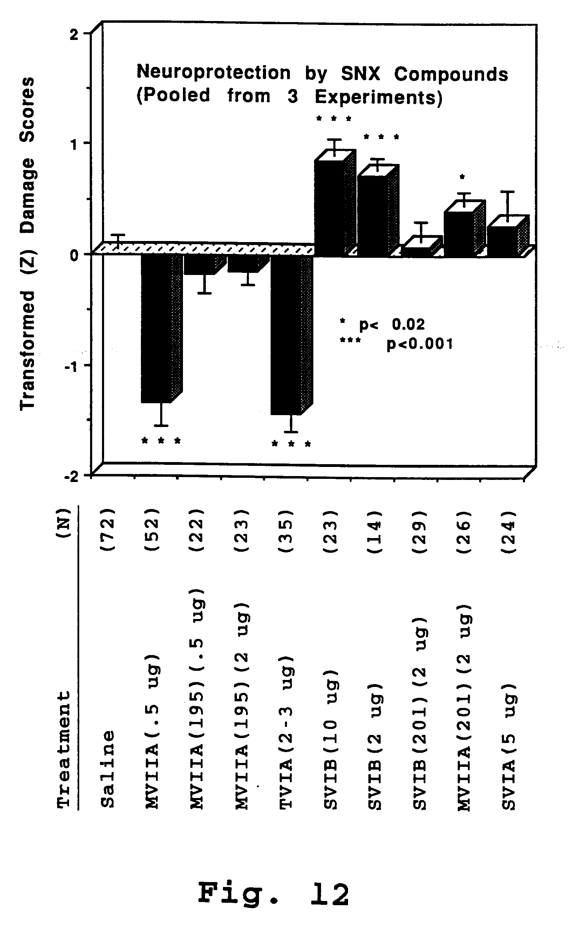

In separate studies, a series of additional OCT peptides were tested in the same animal system. The results of these studies are summarized in Figure 12. Data were pooled from several experiments for this comparison. The data were subjected to Z-score transformation to facilitate comparison between samples having different mean control (saline treatment) damage values. In this analysis, the means of the control groups assume a value of zero, and deviations from the control are shown as positive (indicating an increase in damage compared to controls) and negative (indicating a decrease in damage compared to controls) values. OCT MVIIA and OCT TVIA each showed significant neuroprotection in the studies, as indicated from their significantly negative Z-scores. MVIIA(195), while not significantly different from control, did show a trend toward neuroprotection at the two doses tested, as indicated by its negative Z-scores at both doses. In contrast, OCT SVIB, OCT SVIB(202), OCT MVIIA(201), and OCT SVIA all showed no neuroprotective activity, as indicated by their positive Z-scores.

In a second treatment method, OCT peptide was administered intravenously, as detailed in Example 8B. The degree of neuroprotection in global ischemia produced by OCT MVIIA is indicated in Table 8. "NSD" in the table indicates "not statistically different."

**Table 8**

| Effect of intravenous administration of OCT MVIIA on hippocampal damage in rats | | | | |
|---|---|---|---|---|
| Treatment (mg/kg) | N | Mean Score | SEM | P |
| Saline | 38 | 3.18 | .143 | NSD |
| 1 | 12 | 2.9 | .175 | NSD |
| 3 | 10 | 2.88 | .282 | NSD |
| 5 | 9 | 2.41 | .315 | NSD |
| 15 | 10 | 1.52 | .28 | P< .001 |

In the third ischemia model system, OCT peptide was administered by ICV injection 10 minutes prior to occlusion of the left middle cerebral artery, as detailed in Example 9. Twenty four hours later, the degree of anatomical damage in control and treated animals was examined by magnetic resonance imaging. Eight coronal images were recorded, and the infarct area in each image was determined by counting pixels. Shown in Table 9 is the mean sum of pixels from eight coronal sections per rat. Treatment with 1.7 ug of OCT MVIIA (ICV) resulted in a 24% reduction in the area of the mean infarct size produced by middle cerebral artery occlusion. This reduction was statistically significant, as assessed by the Mann-Whitney U test.

**Table 9**

| Effect of OCT MVIIA on Infarct Size | | | | |
|---|---|---|---|---|
| Treatment (ug/rat) | N | Infarct Size Mean (SEM) | Percent Control | Statistical Significance |
| 0 | 6 | 5211 (293) | 100 | - |
| 0.6 | 6 | 4459 (327) | 87 | 2p>.05 |
| 1.7 | 4 | 3879 ( 99) | 76 | 2p<.01 |

### B. Functional Activity Protection: Hyperactivity

One common consequence of cerebral ischemia in animals is hyperactivity, which can be seen as pacing (exploratory) behavior within a few hours of occlusion, and can be observed up to several days later. Hyperactivity in ischemic gerbils, was monitored as described in Example 10. Briefly, gerbils were tested individually for 60 min, with cumulative activity counts recorded every 15 min for statistical analysis. Baseline activity was measured before surgery to ensure comparability of the different treatment groups on this measure, and activity measurements were made at 1 and 3 days after occlusion.

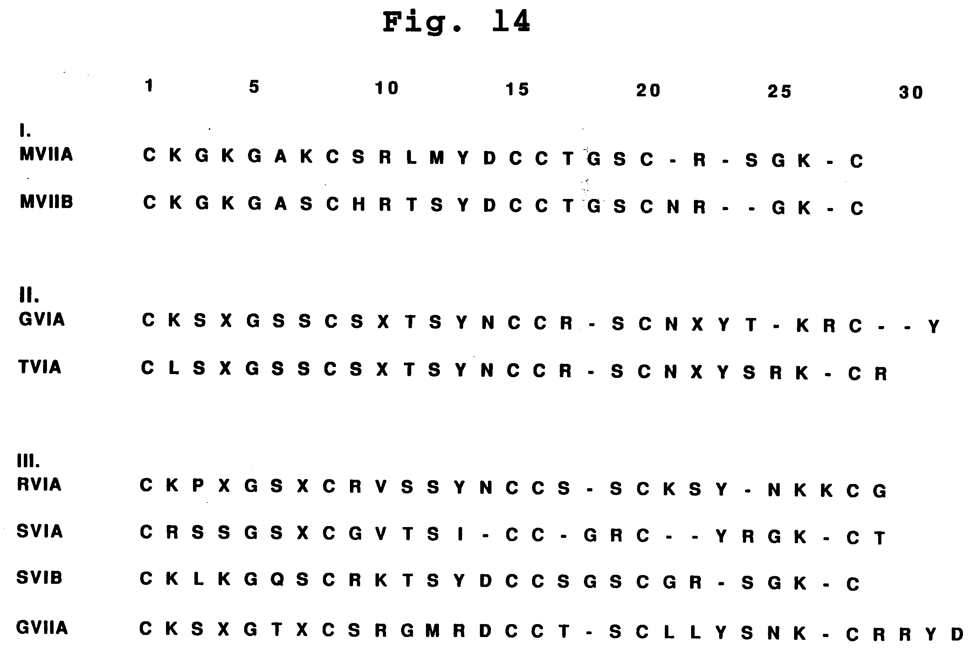

The results of the tests are plotted in Figure 13. The downward slope in each test curve is due to the decrease in activity over the four 15 minutes intervals of the test (1-4 for baseline, 5-8 at day 1, and 9-12 at day three), as the animal becomes more familar with the test environment. Occlusion alone (open triangles) produced a significant rise in activity level over baseline levels 1 day after occlusion, and an elevated activity level was observed over a three-day period, indicating permanent behavioral damage. Non-occluded control animals receiving ICV administration of vehicle (open circles) remained at baseline activity levels through the test period. OCT peptide itself, in the absence of ischemia (solid circles) reduced activity, and this effect persists slightly even at three days. Occluded animals which had been treated with OCT MVIIA (solid triangles) showed lower-than baseline values at 1 day, apparently reflecting the reduced activity produced by the peptide alone. At three days, treated animals showed near-normal levels of activity, indicating that the OCT peptide treatment provided protection against ischemia-induced hyperactivity.

### B. Functional Activity Protection: Spontaneous Alternation

Damage to the hippocampal region of the brain is known to produce deficits in spatial learning and memory, and therefore it could be expected that ischemic damage to hippocampal cells, as documented above, might also be accompanied by loss of functional activity related to short-term memory.

One test which has been widely applied as a measure of short-term memory in experimental animals is the Y maze, in which animals are placed at the base of the stem of a Y "maze", and allowed to enter either of the two Y arms. When the the animal enters an arm, a door is shut behind it. After 5 sec, the animal is returned to its home cage for an intertrial interval (ITI) of 2 to 12 min. At the end of that interval the animal is run in the maze again in the same way. Most normal animals will alternate, that is, will enter the arm that was not entered on the first trial. The test is scored by a "Y" for alternation and an "N" for repeat selection of the same Y arm.

In the test procedure, ischemia in gerbils was induced as above, with simultaneous ICV administration of vehicle (control) or 0.1 or 0.3 µg OCT MVIIA or GVIA peptide (results from all drug treatments were combined, as described in Example 10). Three days after occlusion, the animals were tested in the Y maze. Results of the spontaneous alternation tests are summarized in Table 10 for animals for which there was anatomical protection from doses of at least 0.1 µg of either compound.

**Table 10**

| No. Gerbils Alternating (Y ) or Repeating (N) Experiment Number | | | | | | | | | | | |
|---|---|---|---|---|---|---|---|---|---|---|---|
| | | 3 | | 4 | | 5 | | 6 | | Combined | |
| Ischemia | Drug* | Y | N | Y | N | Y | N | Y | N | Y | N |
| No | No | 9 | 3 | 2 | 2 | 3 | 3 | 6 | 2 | 20 | 10 |
| | Yes | - | - | 4 | 0 | 5 | 1 | 4 | 0 | 13 | 1 |
| Yes | No | 2 | 6 | 4 | 4 | 4 | 4 | 3 | 4 | 13 | 18 |
| | Yes | 4 | 3 | 5 | 2 | 7 | 1 | 7 | 4 | 23 | 10 |

| | | | | | | | | | | | |
|---|---|---|---|---|---|---|---|---|---|---|---|
| * Drug doses are from 0.1 to 0.3 µg of MVIIA or GVIA. | | | | | | | | | | | |

As seen from the data in the table, the normal Y/N ratio for control animals (no occlusion, ICV administration of vehicle) was about 2:1. Ischemic injury produced a drop in this ratio to less than 1, indicating substantially random behavior in the Y test. The loss of short-term memory seen in ischemic animals was completely prevented by peptide treatment, with Y/N ratios of about 2:1 being obtained. Peptide alone in the absence of ischemic injury appeared to enhance the Y/N ratio, and this enhancement may contribute to the improved performance of treated, ischemic animals.

In summary, ischemic animals in which OCT peptide treatment was shown to significantly reduce anatomical damage, also showed statistically improved functional activity, as evidenced by peptide protection against ischemia-induced hyperactivity and loss of short-term memory.

### IV. Neuroprotective OCT Peptide Compounds

### A. Selection of OCT Peptides

Based on a sequence homology analysis of the peptides whose full sequences are known (Figure 1), the naturally occuring neuroprotective OCT peptides were grouped into distinct groups I and II, each with internal homologies distinct to that group, as can be appreciated from Figure 14. Group I includes active OCT peptide MVIIA and MVIIB which possesses a binding constant to the MVIIA site within the range of compounds with neuroprotective activity. Group II includes neuroprotective peptides GVIA and TVIA. A third group includes inactive peptides SVIA and SVIB and OCT peptides whose binding activities for the MVIIA site on neuronal membranes and/or activity in norepinephrine inhibition is outside the range of active compounds.

The three groups of OCT peptides are arranged in Figure 14 with their six Cys residues aligned, which places these residues at positions 1, 8, 15, 16, 20, and 28. To make this alignment, gaps were introduced at the positions shown in the three groups. In the analysis below, these gaps retain the assigned number shown in Figure 14, even though they represent amino acid deletions in the respective groups of active OCT peptides.

Sequence variation in the peptides, based on primary structure alone, was analysed by adopting the following constraints:
1. The peptides in both groups include the Cys residues at position 1, 8, 15, 16, 20, and 28. Other Cys residues could be substituted at the positions indicated below only if they are selectively protected during oxidation of the peptide to form the three disulfide linkages.
2. The peptides in both groups include three disulfide linkages connecting the Cys residues at positions 1 and 16, 8 and 20, and 15 and 30. As described above, the disulfide bridges are formed by air oxidation of the full sequence peptide in the presence of DTT. The ability of the peptide to form the three desired disulfide linkages would therefore require that the peptide, prior to disulfide bridging, be able to adopt a conformation which allows the three selected linkages, with or without the Cys protecting-group strategy discussed above. This constraint would thus exclude amino acid variations which prevent or otherwise hinder the formation of the three selected bridges.
   Constraints 1 and 2 preserve the basic conformation of the OCT peptides imposed by the three disulfide bridges.
3. Within the first group, the amino acid variations which occur at the six non-conserved residues are allowed, including peptides in which the carboxy terminal is amidated or has a free acid form. That is, the first group compounds include the peptide structures having the form:
   CKGKGAX₁CX₂RX₃X₄YDCCTGSCX₅RX₆GKC-t,
   where X₁=K or S; X₂=S or H; X₃=L or T; X₄=M or S; X₅= N or a deletion; X₆=S or deletion; and t is a carboxy or amidated carboxy terminal group.
4. Within the second group, the amino acid variations which occur at the five non-conserved residues are allowed, including peptides in which the carboxy terminal is amidated or has a free acid form. Thus, the second group compounds include the peptide structures having the form:
   CX₁SXGSSCSXTSYNCCRSCNXYX₂X₃X₄CX₅-t, where X₁=K or L; X₂=T or S; X₃=K or R; X₄=R or K; X₅=Y or R; and t= a carboxy or amidated carboxy terminal group.
5. Considering both active groups together, amino acid positions which are conserved in all active species are preserved. Thus, for example, the Cys residues, the 5-position glycine, the 13-position tyrosine, the 19-position serine, and the 26-position lysine are all preserved.
6. Considering both active groups together, there are amino acid positions which are likely to be variable within the range of active species. For example, the position 2 amino acid may be lysine or leucine, and the position-3 amino acid may be glycine or serine. In addition, if the two or more amino acids at a variant position are in a common substitution class, substitution within that class may be favorable. Standard substitution classes are the six classes based on common side chain properties and highest frequency of substitution in homologous proteins in nature, as determined, for example, by a standard Dayhoff frequency exchange matrix (Dayhoff). These classes are Class I: Cys; Class II: Ser, Thr, Pro, 4Hyp, Ala, and Gly, representing small aliphatic side chains and OH-group side chains; Class III: Asn, Asp, Glu, and Gln, representing neutral and negatively charged side chains capable of forming hydrogen bonds; Class IV: His, Arg, and Lys, representing basic polar side chains; Class V: Ile, Val, and Leu, representing branched aliphatic side chains, and Met; and Class VI: Phe, Tyr, and Trp, representing aromatic side chains. In addition, each group may include related amino acid analogs, such as ornithin, homoarginine, N-methyl lysine, dimethyl lysine, or trimethyl lysine in class IV, and cyclohexylalanine or a halogenated tyrosine in Group VI. Further, the classes may include both L and D stereoisomers, although L-amino acids are preferred for substitutions.
7. Considering the known inactive species, substitutions to amino acids which are present in inactive species, but not active ones, at any selected residue position, are not favored to preserve activity in the active compounds. Thus, for example, although a 3-position serine is present in both active and inactive compounds, a 4-position serine is present in an inactive species only, and is thus disfavored.

The above amino acid selection rules 6-7 are intended as a guide for allowed amino acid substitutions within neuroprotective OCT peptides. Once an amino acid substitution or modification is made, the peptide is further screened for the requisite calcium channel antagonist activity, and the requisite activities for inhibition of norepinephrine release and binding to the MVIIA binding site of neuronal membranes, as described above.

Several of the amino acid substitutions or modifications to the OCT peptide illustrate the principles outlined above. For example, with reference to Figure 2, the MVIIA(195) compound contains a Lys to Ala substitution at the position corresponding to position 26 in the MVIIA structure shown in Figure 14. Since this substitution is at a conserved-sequence position, it is predicted that the neuroprotective activity would be lost or reduced. As seen above (Figure 12), the MVIIA(195) peptide shows retention of MVIIA binding activity, but reduced norepinephrine activity, and weak neuroprotective activity compared with the unsubstituted MVIIA OCT.

As another example, the MVIIA(201) compound contains substitutions at positions 9-12 from Ser-Arg-Leu-Met to Arg-Lys-Thr-Ser, the sequence at positions 9-12 in the inactive SVIB OCT peptide. The position-9 substitution is not favored since Arg is present at this position in a non-neuroprotective compound, but not in one of the neuroprotective OCT peptides. The position-10 substitution is disfavored for the same reason. The position-11 substitution is favored, however, since the Leu to Thr substitution occurs within the neuroprotective peptides. The Met to Ser substitution at position 12 is favored for the same reason. Since the peptide modification contains two disfavored substitutions, it is predicted that the neuroprotective activity would be lost or reduced. As seen above, the MVIIA(201) peptide shows retention of MVIIA binding activity (Table 3), but reduced norepinephrine inhibitory activity (Table 2), and weak neuroprotective activity Compared with the unsubstituted MVIIA OCT (Figure 12).

### B. OCT Peptides

The invention further includes the active OCT peptides formed according to amino acid selection rules 3 and 4 above, excluding the natural C-terminal amidated OCT peptides MVIIA, MVIIB, GVIA, and TVIA. More specifically, the peptide compounds of the invention have the form:
CKGKGAX₁CX₂RX₃X₄YDCCTGSCX₅RX₆GKC-t (Group 1)
where X₁=K or S; X₂=S or H; X₃=L or T; X₄=M or S; X₅= N or a deletion; X₆=S or deletion, and t= a carboxy or amidated carboxyterminal group, excluding the peptides in which X₁=K, X₂=S, X₃=L, X₄=M, X₅=deletion, and X₆=S; and X₁=S, X₂=H, X₃=T, X₄=s, X₅=N, and X₆=deletion; and
CX₁SXGSSCSXTSYNCCRSCNXYX₂X₃X₄CX₅-t (Group 2)
where X₁=K or L; X₂=T or S; X₃=K or R; X₄=R or K; and X₅=Y or R, excluding the peptides in which X₁=K, X₂=T, X₃=K, X₄=R, and X₅=Y; and X₁=L, X₂=S, X₃=R, X₄=K, and X₅=R.

These peptides are intended for formulation with a suitable pharmaceutical carrier, in the composition of the invention.

### V. Selecting Neuroprotective Compounds

The compound-test methods discussed in Section II can be used, also in accordance with the present invention, to identify calcium channel antagonist compound which have neuroprotective activity. In the screening method, a calcium channel antagonist compound is screened for its ability to inhibit norepinephrine release in mammalian CNS neuronal cells, and for its affinity to neuronal-cell omega-conotoxin MVIIA binding site. The calcium channel antagonist activity of the test compound may be known, or may be shown, e.g., by its ability to inhibit depolarization-evoked calcium channel currents in neuronal cells, as described in Example 1.

The test compound is selected for use in treating such neuronal damage if the compound:
(i) is effective in inhibiting inhibiting norepinephrine release in mammalian CNS neuronal cells, in concentration ranges within which OCT peptides MVIIA, GVIA, and TVIA effectively inhibit such norepinephrine release, and
(ii) has a binding affinity for the OCT MVIIA binding site which is within the range of binding affinities for the binding site of OCT peptides MVIIA, GVIA, and TVIA.

The compounds which can be screened include, in addition to OCT peptides, and analogs and fragments thereof, other peptide and peptide fragments and organic molecules. Preferred screening methods are described above, and detailed in Examples 2, 4, and 5.

The following examples are intended to illustrate various characteristics of the compositions of the invention, and their use in reducing neuronal damage in ischemia-related injury.

### Example 1

### Calcium-Channel Antagonist Activity: Inhibition of Ionic Currents

Ionic currents through calcium channels were examined in cells that were voltage-clamped by a single patch-clamp electrode. These whole-cell patch-clamp studies were performed mainly on N1E115 mouse neuroblastoma cells, although a variety of cell types have been examined.

### A. Current Measurement Methods

Most measurements were obtained using a bath saline that allowed examination of the calcium currents in the absence of other ionic currents. These solutions contained 80 mM NMDG (as a sodium replacement), 30 mM TEACl (to block potassium currents), 10 mM BaCl₂ (as a charge-carrier through the calcium channels), and 10 mM HEPES at pH 7.3. Some solutions also contained 2 mM quinidine (to block potassium currents) and 3 µM tetrodotoxin (to block sodium currents). Normal bath saline was (mM): 140 NaCl, 10 glucose, 3 KCl, 2 CaCl₂, 1 MgCl₂, 10mM HEPES pH 7.3. Intracellular solutions contained 150 mM CsCl, 0.5 mM CaCl₂, 5 mM EGTA, 5 mM MgCl₂, 2 mM K₂ATP at pH 7.3-7.4. Bath saline and all internal solutions were filtered before use.

Pipets were made from Corning 7052 glass (Garner Glass Company, Claremont, CA 91711), coated with Sylgard (Dow Corning, Midland, MI 48640) and fire-polished before use. Bubble numbers were typically 5 to 6, with pipet resistances typically 2-5 MOhms. Corning 8161, Kimble, and other glasses were also used without noticeable effect on the calcium currents observed.

Recordings were carried out at room temperature with an Axopatch 1-C amplifier (Axon Instruments, Foster City, CA 94404) and analyzed with pCLAMP software (Axon Instruments). Data were filtered at 1000 Hz for a typical sampling rate of .1 kHz; in all cases data was filtered at a frequency at most 1/5 of the sampling rate to avoid aliasing. Data were collected on-line by the software. Analysis was performed on-screen with print-out via a Hewlett-Packard LaserJet Printer (Hewlett-Packard, Palo Alto, CA 94306).

The typical experiment was conducted as follows: after seal formation followed by series resistance compensation and capacitative transient cancellation, a voltage clamp protocol was performed wherein the cell potential was stepped from the holding potential (typically -100 mV) to test potentials that ranged from -60 mV to +20 mV in 10 mV increments. The cell was held at the holding potential for 5 seconds between pulses. Protocols starting from other holding potentials usually covered the same range of test potentials.

### B. Current Inhibition Measurement

Figure 3 shows calcium current traces from an N1E-115 mouse neuroblastoma cell. The figure is read from left to right in time, with downward deflections of the trace indicating positive current flowing into the cell. Currents were elicited by a voltage step from 100 mV to -10 mV. The cell was bathed in saline with sodium replaced by NMDG and 10 mM Ba instead of 2 mM Ca. Potassium currents were blocked by TEA in the bath and Cs in the pipet solution.

The three traces in Figure 3, labeled B-D, show decreasing calcium currents, with increasing MVIIA OCT peptide concentrations of 10 nM (3B), 50 nM (3C), and 200 nM (3D).

The response of voltage-gated calcium current to increasing dosages of OCTs MVIIA and GVIA are shown in Figure 4. The calculated IC₅₀ is 12 nM for GVIA and 116 nM for MVIIA. These values indicate extremely high specificity of the peptides for their site of action.

Table 1 compares IC₅₀ values for GVIA, MVIIA, SVIB and SVIA OCTs. Whereas OCT GVIA and OCT MVIIA show 50% inhibition of the measured calcium current at nanomolar concentration range, IC₅₀ values for OCT SVIB and OCT SVIA were not measurable within the range of concentrations tested, and are therefore listed as having IC₅₀ values above the micromolar concentrations indicated. OCT SVIB and OCT SVIA are considered to be inactive in this assay.

### Example 2

### Inhibition of Neurotransmitter Release

### A. [³H]Norepinephrine release from rat hippocampal slices

Male Sprague-Dawley rats were lightly anesthetized with ether, decapitated, and the brains removed. The hippocampi were then dissected free of cerebral cortex and rinsed with room temperature oxygenated uptake buffer (0.1% bovine serum albumin (BSA) and in mM: NaCl, 123, KCl, 4.8; CaCl₂, 1.2; MgSO₄, 1.2; KH₂PO₄, 1.2; glucose, 11; NaHCO₃, 25). Slices (200 or 400 uM thick) were made using a McIlwain Tissue Chopper and were immediately transferred to room temperature uptake buffer. Slices were then distributed to individual wells of a 96-well plate (Dynatech) containing 0.1 ml uptake buffer per well. [3H]Norepinephrine (3 uCi/ml) diluted in uptake buffer containing 1 mM ascorbate and test compound was then added to each well. Incubation was at 37 degrees for 30 minutes in a humidified, 5% CO₂ incubator. Bathing buffer was then removed and slices washed two times for 11 minutes each with basal buffer containing appropriate test compound (basal buffer: 0.1 % BSA and in mM: NaCl, 123, KCl, 5.0; CaCl₂, 0.4; MgSO₄, 1.2; KH₂PO₄, 1.2; glucose, 11; NaHCO₃, 25). Each slice was then incubated for 15 minutes in 0.1 ml of basal buffer. This buffer was then removed for measurement and replaced by 0.1 ml stimulation buffer (0.1 % BSA in mM:NaCl, 97, KCl, 30; CaCl2, 0.4; MgSO₄, 1.2; KH₂PO₄, 1.2; glucose, 11; NaHCO₃, 25) for 15 minutes. Stimulation buffer was then removed for measurement of radioactivity. Radioactivity remaining in each slice was determined. Data were normalized to total cpm of radioactivity per slice: $\text{total radioactivity = S + B + slice}$, where S is the amount of radioactivity present in the stimulation buffer, and B is the amount of radioactivity present in the basal buffer. Stimulated release, as a percentage of $\text{total radioactivty - 100 (S/(S+B+slice))}$, and basal release, as a percentage of $\text{total radioactivity = 100 (B/(S+B+slice))}$. Concentration effect graphs are plotted as in Figure 5. Computer aided curve fitting was used to determine IC₅₀ values from such data. These values are given in Figure 2.

### Example 3

### Synaptosomal Membrane Preparations

### A. Mammalian-Brain Synaptosomes and Synaptosomal Membranes

Synaptosomes were prepared from rat whole brain or hippocampal region of brain. Rats were sacrificed, and forebrains were removed and transferred to 10 ml ice-cold 0.32 M sucrose containing the following protease inhibitors (PI): 1 mM EGTA; 1 mM EDTA; 1 uM pepstatin; 2 uM leupeptin. Brains were homogenized using a motor-driven Teflon-glass homogenizer (approx. 8 passes at 400 rpm). Homogenates from 4 brains were pooled and centrifuged at 900 xg for 10 minutes at 4 degrees. Supernatants were then centrifuged at 8,500 xg for 15 minutes. Resulting pellets were resuspended in 10 ml each ice-cold 0.32 M sucrose plus PI with vortex mixing. The suspension was then centrifuged at 8,500 xg for 15 minutes. Pellets were resuspended in 20 ml ice-cold 0.32 M sucrose plus PI. The suspension (5 ml/tube) was layered over a 4-step sucrose density gradient (7ml each: 1.2 M sucrose, 1.0 M sucrose, 0.8 M sucrose, 0.6 M sucrose; all sucrose solutions Containing PI). Gradient tubes were centrifuged in a swinging bucket rotor at 160,000 xg for 60 minutes at 4 degrees. The 1.0 M sucrose layer plus the interface between the 1.0 and 1.2 M sucrose layers were collected and diluted with ice cold deionized water plus PI to yield a final sucrose concentration of 0.32 M. The resulting suspension was centrifuged at 20,000 xg for 15 minutes. Pellets were then resuspended in 5 ml ice-cold phosphate buffered saline plus PI. The resulting rat brain synaptosomes were then aliquoted and stored in a liquid nitrogen containment system.

Prior to use in binding assays, synaptosomes were thawed and diluted with 3 volumes of ice cold deionized water plus PI. This suspension was homogenized using a PT 10-35 Polytron (setting 6) for two 10-second bursts. The homogenate was centrifuged at 40,000 xg for 20 minutes at 4 degrees. The resulting pellets were resuspended in about 5 ml of ice cold phosphate buffered saline plus PI. The resulting brain synaptosomal membrane preparation was aliquoted and stored at -80 °C until use. Protein concentration of the membrane preparation was determined using Bradford reagent (BioRad), with bovine serum albumin as standard.

### Example 4

### OCT Peptide Binding to MVIIA Binding Site in Synaptosomal Membranes

### A. Saturation Binding Assay

MVIIA OCT was radiolabeled with ¹²⁵I-iodine by reaction with IodogenTM, essentially according to the method of Ahmad et al. Following the Iodogen reaction, the peptide solution was chromatographed by HPLC through a C-8 reverse phase column and eluted with a gradient from 0.1% trifluoroacetic acid to 60% acetonitrile in 0.1% trifluoroacetic acid. The major peak of radioactivity following the underivatized MVIIA OCT was collected.

The binding constant (K_{d}) for [¹²⁵I]-MVIIA OCT to rat brain synaptosomal membranes was determined by a saturation binding method in which increasing quantities of [125I] MVIIA OCT were added to aliquots of a synaptosomal membrane preparation (10 ug membrane protein, suspended in binding buffer consisting of 20 mM HEPES, pH 7.0, 75 mM NaCl, 0.1 mM EGTA, 0.1 mM EDTA, 2µM leupeptin, .035 µg/ml aprotinin, and 0.1% bovine serum albumin (BSA), in a total volume of 0.5 ml). Binding at each concentration of labeled compound was determined in the absence and presence of 1 nM unlabeled MVIIA OCT to determine specific binding. The amount of labeled peptide specifically bound at each concentration was used to determine Bₘₐₓ, the concentration of specific binding sites on the synaptosomes, and K_{d}, following standard binding analysis methods (Bennett). Figure 6A shows a saturation binding curve of [¹²⁵I]MVIIA to rat synaptosomal membranes. Figure 6B shows a Scatchard transformation of the data, from which a calculated Kd of about 10 pM is determined.

### B. Competitive Displacement Binding Assay

Rat brain synaptosomal membranes prepared as described in Example 3 were suspended in a binding buffer consisting of 20 mM HEPES, pH 7.0, 75 mM NaCl, 0.1 mM EGTA, 0.1 mM EDTA, 2µM leupeptin, .035 µg/ml aprotinin, and 0.1% bovine serum albumin (BSA). [¹²⁵I]-MVIIA OCT (25-30,000 cpm, approximately 1500-2000 Ci/mmol) and test compound were aliquoted into polypropylene tubes, in the absence or presence of 1 nM MVIIA OCT to determine non-specific binding. The membrane suspension was diluted and aliquoted last into the test tubes, such that each assay tube contained 10 µg membrane protein and the total volume was 0.5 ml. After incubation for 1 hour at room temperature, tubes were placed in an ice bath, then filtered through GF/C filters (Whatman), which were pre-soaked in 0.6% polyethyleneimine and prewashed with wash buffer (20 mM HEPES, pH 7.0, 125 mM NaCl, 0.1% BSA) using a Millipore filtration system. Just prior to filtration, each assay tube received 3 ml ice-cold wash buffer. The filtered membranes were washed with two 3-ml volumes of ice-cold wash buffer, dried, and filter-bound radioactivity was measured in a Beckman gamma counter (75% counting efficiency).

Representative displacement binding curves for rat brain synaptosomal membranes are illustrated in Figure 7. IC₅₀ values were computed from line fit curves generated by a 4-parameter logistic function. These values represent the concentration of test compound required to inhibit by 50% the total specific binding of [¹²⁵I] MVII-OCT to rat brain synaptosomal membranes, where specific binding is defined as the difference between binding of [¹²⁵I] MVIIA OCT in the absence and presence of excess (1 nM) unlabelled [¹²⁵I] MVIIA OCT. Such values serve as approximations of the relative affinities of a series of compounds for a specific binding site.

The binding constant (Kᵢ) for each test substance was calculated using non-linear, least-squares regression analysis (Bennett & Yamamura) of competitive binding data from 2 assays performed in duplicate on separate occasions. The relationship between Ki and IC₅₀ (concentration at which 50% of labeled compound is displaced by test compound is expressed by the Cheng-Prusoff equation:${\text{K}}_{\text{i}} {\text{= IC}}_{\text{50}} \text{/(1 +[L]/Kd)}$ where IC₅₀ is the concentration of test substance required to reduce specific binding of labeled ligand by 50%; [L] is the concentration of [¹²⁵I]-MVIIA OCT used in the experiment; and K_{d} is the binding constant determined for binding of [¹²⁵I]-MVII OCT to rat brain synaptosomal membranes in saturation binding experiments. Table 3 summarizes computed IC₅₀ and Ki values for various OCT peptides for the MVIIA binding site of rat brain synaptosome.

### Example 5

### Identification of OCT MVIIA Binding Protein

Synaptosomal membranes from rat brain hippocampal region (RHM) were prepared as described in Example 3. An aliquot of synaptosomal preparation containing 40 µg protein was diluted in cross-link binding buffer (20 mM HEPES, pH 7.1, 75 mM NaCl, 0.1 mM EDTA, 0.1 mM EGTA, 0.002 mM leupeptin, 0.5 TIU/ml aprotinin). This suspension was then pelleted (13,000 X g, 15 min.). The pellet was then resuspended in crosslink binding buffer, pelleted again, and the resulting pellet was suspended in crosslink binding buffer to give washed synaptosomal preparations having final protein concentrations of approximately 1 mg/ml.

Binding was carried out in a total volume of 0.5 ml, containing 0.4 ml of appropriate crosslink binding buffer, 0.05 ml of washed synaptosomal preparation (concentration of binding sites: (final Concentration: 0.05 µM), 0.05 ml of [¹²⁵I] MVIIA OCT (final Concentration: 0.1 nM). In separate aliquots, 0.005 ml unlabeled MVIIA OCT was added to assess nonspecific binding (final concentration: 0.05 µM). Incubation was at 20-24° for 25 min., rotating samples end over end.

At the end of the incubation period, crosslinking of bound [¹²⁵I] MVIIA OCT to its receptor was carried out by adding .01 ml of 25 mM 1-ethyl-3-(3-dimethylaminopropyl) carbodiimide HCL (EDC) to the suspension (EDC dissolved in 25 mM PIPES, pH 6.1, immediately prior to use). The mixture was incubated for ten minutes at ice temperature, with intermittent mixing. The reaction was quenched by addition of 20 mM ammonium acetate. The mixture was then pelleted by centrifugation at 13,000 X g for 15 min., and subsequently washed 1-2 times by resuspension in 25 mM HEPES buffer pH 7.5 and pelleting. The final pellet was dissolved in 20 µl fresh sample buffer (200 mM Tris-HCl, 10 mM dithiothreitol, 4 M urea, 8% SDS, 10% glycerol, 0.1% bromphenol blue), then subjected to SDS PAGE (4-15% acrylamide gradient gel) without prior heating of the sample. Similar experiments were carried out, using [¹²⁵I] GVIA OCT as crosslink ligand.

Figure 8 shows an autoradiograph demonstrating the crosslinked binding of [¹²⁵I] MVIIA OCT and [¹²⁵I] MVIIB OCT to rat brain synaptosomal membrane preparations. Although a number of protein bands were labeled by the procedure, binding at a protein band migrating as a 200-230 Kdaltons protein was specifically displaced by inclusion of excess unlabelled ligand as described above.

These results suggest that high affinity specific binding to rat synaptosomal membranes is at least in part attributable to binding at a protein band which migrates in the 200-230 Kd region on a gel. Labeling of other bands may be due to either lower affinity or non-specific binding, since labeling to these bands was not displaced by unlabeled ligand.

### Example 6

### OCT Peptide Binding to SVIB Binding Site in Synaptosomal Membranes

Rat brain synaptosomal membranes were prepared as described in Example 3. OCT SVIB was radiolabeled by iodination with ¹²⁵I-iodine by the Iodogen reaction, described in Example 4. Displacement binding of radiolabeled SVIB on rat brain synaptosomal membranes was carried out as in Example 4B. SVIB displacement curves for several of the OCT peptides assayed is shown in Figure 9. IC₅₀ values and Kᵢ values were calculated as described in Example 4. Table 4 shows the calculated Kᵢ values for the OCT peptides examined, and the ratio of Kᵢ binding constants to the OCT MVIIA site and to the SVIB binding site.

### Example 7

### Reduction in Anatomical Damage: Global Ischemia Model 1

Global ischemic damage was examined in the gerbil model, according to standard procedures (Kirino). Male mongolian gerbils (Meriones unguiculatus, Tumblebrook Farm, West Brookfield, MA) weighing 50-80 g were anesthetized in a small chamber with 4% halothane carried by 70% nitrous oxide (0.44 L/min) and 30% oxygen (0.19 L/min). They were then maintained throughout surgery with 2% halothane by placing their noses through a hole in a rubber dam on a gas delivery tube. Using aseptic techniques, both common carotid arteries were exposed, dissected free of surrounding tissue, and occluded with microvascular clamps approximately 3 to 4 mm above the clavicle. The occlusions were maintained for 8 minutes, timed while both arteries were occluded. There was generally a period of approximately 1 minute between clamping of each of the two arteries, and approximately 4 seconds between unclamping them. After the clamps were removed, the skin was sutured shut and anesthesia discontinued.

During or after the occlusion, an intracerebroventricular (ICV) injection aimed at the lateral ventricle was made. To accomplish this, a 10 microliter Hamilton syringe with a 27 gauge needle was filled with injectate by backloading to assure the absence of air in the system. A stiff plastic sleeve was slipped onto the needle so that 3.5 mm of the needle protruded past the sleeve. The skull around the bregma was exposed, a distance of 1.1 mm left of the midline was measured with a compass, and a distance of 0.4 mm posterior to bregma was approximated by eye. The needle tip was held perpendicular to the skull and inserted through it at that point by applying gentle pressure while twisting. It was advanced until the sleeve abutted the skull, and 5 microliters of injectate was infused over a period of approximately 3 sec. The skin was then sutured shut. Occluded animals received either drug or its vehicle. Injected, unoccluded controls were anesthetized, and received the ICV injection only.

Animals were anesthetized with CO₂. The chest cavity was opened and the animal was perfused through the heart with approximately 3 milliliters of phosphate-buffered saline (PBS; 0.10 M sodium phosphate; 0.15 M sodium chloride) containing heparin (10 Units/ml), followed by approximately 10 ml of Zamboni's fix (15% (vol/vol) picric acid 4% (wt/vol) paraformaldehyde in 0.1 M phosphate buffer pH 7.4 or 10% phosphate buffered formalin. Brains were removed and left immersed in the same fixative for several hours.

Brains were blocked just posterior to the optic chiasm and posterior to the mammillary bodies. They were then placed in 10% (wt/vol) sucrose in PBS overnight at 4 degrees. The block containing the hippocampus was frozen with liquid Freon onto a cryostat chuck using Tissue-Tek^{R} O.C.T. embedding medium for frozen tissue specimens (Miles Inc., Elkhart, IA). Sections 10 microns in thickness were cut. Series of 5 sections were collected, with each series approximately 100 microns apart, until the relevant part of the hippocampus was obtained (40-50 sections per brain). At least 8 sections per brain were stained with hematoxylin and eosin, substantially according to reported procedures.

Coverslips were then placed over the sections, using Permount™ as an adhesive. Figures 10A and 10B are low-power micrographs of gerbil hippocampus (CA1 region) in animals after ischemia, after infusion of MVIIA OCT (10A) or after drug vehicle (10B). The arrows in the figures indicate the approximate borders of the CA1 region of the hippocampus. At higher power, cells in the drug-treated ischemic animals appear normal (Figure 11A), whereas damage is apparent in the ischemic animals receiving vehicle alone (Figure 11B). Another example of complete drug protection is seen in Figure 11C, and an example of partial protection is seen in Figure 11D, where there are a small number of damaged cells.

Sections, such as those seen in Figures 10 and 11, were viewed and scored by an investigator having no knowledge of the treatment of any particular sample. Ischemic damage was scored in the CA1 region of the hippocampus. Damage was generally seen as pink (eosinophilic) cytoplasm and shrunken, dark blue nuclei. Scoring was as described below:

| Score | Observation |
|---|---|
| 0 | No damaged cells were apparent. |
| 1 | Less than 25% damaged cells in a CA1 field, or damage was restricted to the extreme edges of the CA1 region. |
| 2 | Approximately 50% damaged cells in a CA1 field, or damage to less than half the length of CA1. |
| 3 | Damaged cells outnumber normal cells to a maximum of 75%, with damage extending throughout most of CA1. |
| 4 | Complete damage to CA1, with fewer than 25% normal cells surviving. |

The extent of anatomical damage in ischemic animals treated with MVIIA or GVIA OCT or receiving vehicle alone (control), based on the above scoring system, is given in Table 5. The peptide was administered by ICV infusion during the eight minutes of occlusion, at a total dose indicated in Table 5. As seen, the extent of damage in the higher-dose MVIIA OCT treated animal was only 25% of that in untreated animals. The GVIA peptide also produced more than a 50% reduction in damage, and the lower dose was near maximal effectiveness.

In a second treatment method, the OCT peptide was administered by ICV infusion 1 hour after the 8-min occlusion, at the same drug dosage level as indicated above. The anatomical damage in the presence and absence of drug, scored as above, is given in Table 6.

### Example 8

### Reduction in Anatomical Damage: Global Ischemia Model 2

Global ischemic damage was examined in the rat brain model, employing the four-vessel occlusion method of Pulsinelli and Brierly (Pulsinelli) for introducing temporary global ischemia in rats. Although the two carotid arteries supply blood to the forebrain, their occlusion alone has only moderate effects on forebrain blood flow because the posterior communicating arteries allow blood to be shunted from the brainstem blood supply, which is fed by the two vertebral arteries. Therefore, in order to effect severe forebrain ischemia, all four vessels must be occluded. The procedure used allows ischemia to be produced in conscious animals, by closing surgically implanted clamps, and therefore avoid possible interactions with drug treatment. The procedure was modified to allow carotid occlusion without the need for reopening a skin wound in conscious animals.

Surgery was performed to permanently occlude both vertebral arteries and to implant an arterial clasp to allow temporary occlusion of the carotid arteries at a later time. Under sodium pentobarbital anesthesia (60 mg/kg) male Fisher 344 rats were placed in a stereotaxic holder and the first cervical vertebra was exposed with the aid of a dissecting microscope. The vertebral arteries were occluded through the alar foramina with a thermocautery device and the skin closed with wound clips. The animal was placed on its back and the carotid arteries were carefully dissected free of the surrounding nerves and vessels under the microscope. The loose end of the Silastic loop of the clasp was passed behind the artery and put through the open side of the clasp and secured as for the other end. This was then repeated for the other carotid. The clasps were tied into the skin with 3-0 suture as the skin was closed so as to externalize the ends of the loop.

Ischemia was produced 2 days after surgery. To occlude the carotid arteries, the animal was held by lightly pinching the skin at the back of the neck and the ends of each loop were pulled out and secured with a bulldog clamp. At the end of the 15 min. occlusion, the clamps were removed to allow reperfusion. An effective occlusion causes the animal to lose its righting response within about 1 min. of occlusion. When the animal did not lose the righting response or regains it during occlusion, the loops were pulled tighter to assure complete carotid occlusion. Animals that did not lose their righting response were eliminated from the study, because this suggested that there was still significant cerebral blood flow.

Neuropathological analysis (see below) of such animals `has confirmed this observation, because the damage is found to be less than in animals that do lose their righting response. Some animals righted themselves once or twice during the occlusion but immediately lost the righting response again, and were not eliminated from the study. Any animal that righted itself and remained up was eliminated.

### A. Intracerebroventricular Administration of OCT Peptide

Rats receiving intracerebroventricular (ICV) compound were anesthetized using halothane immediately following reperfusion, and compound contained in 5 µL saline or saline alone was injected into the lateral ventricle as for gerbils. The coordinates of the injection were 1.2 mm left of midline and 0.5 mm posterior to bregma, at a depth of 3-4 mm. Rectal temperature was monitored from just before occlusion, and for 4-6 hours post occlusion. Rats were maintained normothermic (rectal temperature at about 37 degrees) for 4-6 hours following occlusion, by means of heating apparatus. The degree of neuroprotection is shown in Table 7.

### B. Intravenous Administration of OCT Peptide

For intravenous (IV) administration of compound in conjunction with the rat 4-VO model of cerebral ischemia, rats were subjected to surgery and subsequent occlusion as described above. Following removal of occlusion clamps, rats were placed into Rodent Restraint Cones (Harvard Bioscience). Reversible tourniquets were applied to tail veins, and OCT MVIIA was injected in a total volume of 0.25 ml, in the doses indicated in Table 8. As for ICV administration, rats were maintained normothermic (rectal temperature at about 37 degrees) for 4-6 hours following occlusion, by means of heating apparatus. The degree of neuroprotection is shown in Table 8.

### Example 9

### Reduction in Anatomical Damage: Focal Ischemia Model

The rat middle Cerebral artery occlusion model of cerebral ischemia was performed on SHR strain rats. Rats were anesthetized using Evipan (150 mg/kg i.p.). OCT MVIIA was injected in a volume of 5 ul intracerebroventricularly into the left lateral ventricle, as described in Example 8. Within 10 minutes, the left middle cerebral artery was permanently occluded by electrocoagulation. Twenty four hours after the occlusion was performed, rats were again anesthetized with Evipan for magnetic resonance imaging. Eight coronal images were recorded. The infarct area in each image was determined by counting pixels. Shown in Table 9 is the mean sum of pixels from eight coronal sections per rat. Treatment with 1.7 ug of MVIIA OCT (i.c.v) resulted in a 24% reduction in the area of the mean infarct size produced by middle cerebral artery occlusion. This reduction was statistically significant, as assessed by the Mann-Whitney U test.

### Example 10

### Protection Against Loss of Functional Activity

### A. Hyperactivity

One common sequela of cerebral ischemia is hyperactivity, which can be seen as pacing behavior within a few hours of occlusion and can be measured up to several days later. Hyperactivity was quantitated with Automex activity monitors (Columbia Instruments, Columbus, OH), which record perturbations of a radiofrequency field. Gerbils were tested individually in 17x27-cm plastic cages for 60 min, with cumulative activity counts recorded every 15 min for statistical analysis. Baseline activity was measured before surgery to ensure comparability of the different treatment groups on this measure.

The results of the tests are plotted in Figure 13. The downward slope in each test curve is due to the decrease in activity over the four 15 minutes intervals of the test (1-4 for baseline, 5-8 at day 1, and 9-12 at day three), as the animal becomes more familiar with the test environment. Occlusion alone (open triangles) produced a significant rise in activity level over baseline levels 1 day after occlusion, and an elevated activity level was observed over a three-day period, indicating permanent behavioral damage. Non-occluded control animals receiving ICV administration of vehicle (open circles) remained at baseline activity levels through the test period. OCT peptide itself, in the absence of ischemia (solid circles) reduces activity, and this effect persists slightly even at three days. Occluded animals which had been treated with OCT MVIIA (solid triangles) showed lower-than baseline values at 1 day, apparently reflecting the reduced activity produced by the peptide alone. At three days, treated animals showed near-normal levels of activity, indicating that the OCT peptide treatment provided protection against ischemia-induced hyperactivity.

### B. Spontaneous Alternation

Because the predominant neuropathological consequence of the type of ischemia used here is hippocampal damage (Example 7) which is known to produce deficits in spatial learning and memory, a test of recent (working) memory in maze performance were employed. This test uses a Y maze.

Gerbils were tested in a Y maze, in which the animal is placed in the base of the stem of the maze, and when the animal enters an arm, a door is shut behind it. After 5 sec, the gerbil is returned to its home cage for an intertrial interval (ITI) of 2 to 12 min. At the end of that interval the gerbil is run in the maze again in the same way. Most normal animals will alternate, that is, will enter the arm that was not entered on the first trial. Occasionally an animal did not enter an arm within about 1 min. because it had a seizure, so it was eliminated from that test.

Because individual experiments include too few animals per group to allow meaningful statistical evaluation of the data, the results were combined for all experiments in which there was good evidence of protection by drug treatment against hippocampal damage (Example 7). Only experiments with positive results were combined to determine if the anatomical protection was associated with behavioral protection.

Results of the spontaneous alternation tests are summarized in Table 10 for experiments in which there was anatomical protection from doses of at least 0.1 µg of either compound. A chi square test on the combined data was significant at p <0.01. Combining treatment groups to examine each factor separately (e.g., all occluded vs. all unoccluded, regardless of drug treatment) indicated that each was significant by chi square at p <0.05; that is, (a) ischemia caused worse performance and (b) the level of performance was largely restored in treated animals.

## Claims (Claims for the following Contracting State(s): AT, BE, CH, DK, FR, GB, IT, LI, LU, NL, SE)

1. The use of a neuronal calcium-channel antagonist peptide having activities for blocking norepinephrine release in mammalian central nervous system neuronal cells, and for binding to neuronal-membrane omega-conotoxin MVIIA binding site, which are within the range of such activities for omega-conotoxin MVIIA, GVIA, or TVIA, for the manufacture of a medicament for reducing neuronal damage related to an ischemic condition in a mammalian species.

2. The use according to Claim 1, wherein the neuronal calcium-channel antogonist peptide is an omega conotoxin peptide.

3. The use according to Claim 2, wherein the activity for binding is characterized by a ratio of binding constants of the peptide for the neuronal-cell omega-conototoxin MVIIA site and for a neuronal-cell omega-conototoxin SVIB binding site which is within the range of such ratios measured for omega conotoxin peptides MVIIA, GVIA, or TVIA.

4. The use according to Claim 2, wherein the peptide has the form:
CKGKGAX₁CX₂RX₃X₄YDCCTGSCX₅RX₆GKC-t, where X₁=K or S; X₂=S or H; X₃=L or T; X₄=M or S; X₅=N or a deletion; X₆=S or deletion; and t= carboxy or amidated carboxy terminal group.

5. The use according to Claim 2, wherein the peptide has the form:
CX₁SXGSSCSXTSYNCCRSCNXYX₂X₃X₄CX₅-t, where X₁=K or L; X₂=T or S; X₃=K or R; X₄=R or K; X₅=Y or R; and t= carboxy or amidated carboxy terminal group.

6. The use according to Claim 2, wherein the peptide is selected from the group consisting of omega conotoxins MVIIA, GVIA, and TVIA.

7. An omega conotoxin peptide having the form:
CKGKGAX₁CX₂RX₃X₄YDCCTGSCX₅RX₆GKC-t, where X₁=K or S; X₂=S or H; X₃=L or T; X₄=M or S; X₅=N or a deletion; X₆=S or deletion; and t= carboxy or amidated carboxy terminal group, excluding the peptides in which X₁=K, X₂=S, X₃=L, X₄=M, X₅=deletion, and X₆=S; and X₁=S, X₂=H, X₃=T, X₄=S, X₅=N, and X₆=deletion.

8. The conotoxin peptide having the form:
CX₁SXGSSCSXTSYNCCRSCNXYX₂X₃X₄CX₅-t, where X₁=K or L; X₂=T or S; X₃=K or R; X₄=R or K; X₅=Y or R; excluding the peptides in which X₁=K, X₂=T, X₃=K, X₄=R, X₅=Y; and X₁=L, X₂=S, X₃=R, X₄=K, and X₅=R.

9. A method of selecting a neuronal-cell calcium-channel peptide antagonist compound which can be used in the manufacture of a medicament for reducing ischemia-related neuronal damage, such as produced by stroke, in a human, comprising
assaying a compound for its ability to inhibit norepinephrine release in central nervous system neuronal cells, and for its affinity to neuronal-cell omega-conotoxin MVIIA binding site,
selecting the assayed compound for use in preparing such a medicament if the compound:
(i) is effective in inhibiting norepinephrine release in central nervous system neuronal cells, at a concentration within which omega conotoxins MVIIA, GVIA, and TVIA are effective to inhibit such norepinephrine release in neuronal cells, and
(ii) has a binding affinity for such omega-conotoxin MVIIA binding site which is within the range of binding affinities for the binding site of omega conotoxins MVIIA, GVIA, or TVIA.

10. The method of Claim 9, wherein the binding affinity of the compound is characterized by a binding constant measured by competitive displacement by the compound of omega-conotoxin MVIIA from neuronal membranes.

11. The method of Claim 9, wherein the binding affinity of the compound is characterized by a ratio of binding constants of the compound for the neuronal-cell omega-conotoxin MVIIA site and for neuronal-cell omega-conotoxin SVIB binding site.

## Claims (Claims for the following Contracting State(s): DE)

1. The use of a neuronal calcium-channel antagonist peptide having activities for blocking norepinephrine release in mammalian central nervous system neuronal cells, and for binding to neuronal-membrane omega-conotoxin MVIIA binding site, which are within the range of such activities for omega-conotoxin MVIIA, GVIA, or TVIA, for the manufacture of a medicament for treating cerebral ischemia

2. The use according to Claim 1, wherein the neuronal calcium-channel antogonist peptide is an omega conotoxin peptide.

3. The use according to Claim 2, wherein the activity for binding is characterized by a ratio of binding constants of the peptide for the neuronal-cell omega-conototoxin MVIIA site and for a neuronal-cell omega-conototoxin SVIB binding site which is within the range of such ratios measured for omega conotoxin peptides MVIIA, GVIA, or TVIA.

4. The use according to Claim 2, wherein the peptide has the form:
CKGKGAX₁CX₂RX₃X₄YDCCTGSCX₅RX₆GKC-t, where X₁=K or S; X₂=S or H; X₃=L or T; X₄=M or S; X₅=N or a deletion; X₆=S or deletion; and t= carboxy or amidated carboxy terminal group.

5. The use according to Claim 2, wherein the peptide has the form:
CX₁SXGSSCSXTSYNCCRSCNXYX₂X₃X₄CX₅-t, where X₁=K or L; X₂=T or S; X₃=K or R; X₄=R or K; X₅=Y or R; and t= carboxy or amidated carboxy terminal group.

6. The use according to Claim 2, wherein the peptide is selected from the group consisting of omega conotoxins MVIIA, GVIA, and TVIA.

7. An omega conotoxin peptide having the form:
CKGKGAX₁CX₂RX₃X₄YDCCTGSCX₅RX₆GKC-t, where X₁=K or S; X₂=S or H; X₃=L or T; X₄=M or S; X₅=N or a deletion; X₆=S or deletion; and t= carboxy or amidated carboxy terminal group, excluding the peptides in which X₁=K, X₂=S, X₃=L, X₄=M, X₅=deletion, and X₆=S; and X₁=S, X₂=H, X₃=T, X₄=S, X₅=N, and X₆=deletion.

8. The conotoxin peptide having the form:
CX₁SXGSSCSXTSYNCCRSCNXYX₂X₃X₄CX₅-t, where X₁=K or L; X₂=T or S; X₃=K or R; X₄=R or K; X₅=Y or R; excluding the peptides in which X₁=K, X₂=T, X₃=K, X₄=R, X₅=Y; and X₁=L, X₂=S, X₃=R, X₄=K, and X₅=R.

9. A method of selecting a neuronal-cell calcium-channel peptide antagonist compound which can be used in the manufacture of a medicament for reducing ischemia-related neuronal damage, such as produced by stroke, in a human, comprising
assaying a compound for its ability to inhibit norepinephrine release in central nervous system neuronal cells, and for its affinity to neuronal-cell omega-conotoxin MVIIA binding site,
selecting the assayed compound for use in preparing such a medicament if the compound:
(i) is effective in inhibiting norepinephrine release in central nervous system neuronal cells, at a concentration within which omega conotoxins MVIIA, GVIA, and TVIA are effective to inhibit such norepinephrine release in neuronal cells, and
(ii) has a binding affinity for such omega-conotoxin MVIIA binding site which is within the range of binding affinities for the binding site of omega conotoxins MVIIA, GVIA, or TVIA.

10. The method of Claim 9, wherein the binding affinity of the compound is characterized by a binding constant measured by competitive displacement by the compound of omega-conotoxin MVIIA from neuronal membranes.

11. The method of Claim 9, wherein the binding affinity of the compound is characterized by a ratio of binding constants of the compound for the neuronal-cell omega-conotoxin MVIIA site and for neuronal-cell omega-conotoxin SVIB binding site.

## Claims (Claims for the following Contracting State(s): ES, GR)

1. The use of a neuronal calcium-channel antagonist peptide having activities for blocking norepinephrine release in mammalian central nervous system neuronal cells, and for binding to neuronal-membrane omega-conotoxin MVIIA binding site, which are within the range of such activities for omega-conotoxin MVIIA, GVIA, or TVIA, for the manufacture of a medicament for reducing neuronal damage related to an ischemic condition in a mammalian species.

2. The use according to Claim 1, wherein the neuronal calcium-channel antogonist peptide is an omega conotoxin peptide.

3. The use according to Claim 2, wherein the activity for binding is characterized by a ratio of binding constants of the peptide for the neuronal-cell omega-conototoxin MVIIA site and for a neuronal-cell omega-conototoxin SVIB binding site which is within the range of such ratios measured for omega conotoxin peptides MVIIA, GVIA, or TVIA.

4. The use according to Claim 2, wherein the peptide has the form:
CKGKGAX₁CX₂RX₃X₄YDCCTGSCX₅RX₆GKC-t, where X₁=K or S; X₂=S or H; X₃=L or T; X₄=M or S; X₅=N or a deletion; X₆=S or deletion; and t= carboxy or amidated carboxy terminal group.

5. The use according to Claim 2, wherein the peptide has the form:
CX₁SXGSSCSXTSYNCCRSCNXYX₂X₃X₄CX₅-t, where X₁=K or L; X₂=T or S; X₃=K or R; X₄=R or K; X₅=Y or R; and t= carboxy or amidated carboxy terminal group.

6. The use according to Claim 2, wherein the peptide is selected from the group consisting of omega conotoxins MVIIA, GVIA, and TVIA.

7. A method of selecting a neuronal-cell calcium-channel peptide antagonist compound which can be used in the manufacture of a medicament for reducing ischemia-related neuronal damage, such as produced by stroke, in a human, comprising
assaying a compound for its ability to inhibit norepinephrine release in central nervous system neuronal cells, and for its affinity to neuronal-cell omega-conotoxin MVIIA binding site,
selecting the assayed compound for use in preparing such a medicament if the compound:
(i) is effective in inhibiting norepinephrine release in central nervous system neuronal cells, at a concentration within which omega conotoxins MVIIA, GVIA, and TVIA are effective to inhibit such norepinephrine release in neuronal cells, and
(ii) has a binding affinity for such omega-conotoxin MVIIA binding site which is within the range of binding affinities for the binding site of omega conotoxins MVIIA, GVIA, or TVIA.

8. The method of Claim 7, wherein the binding affinity of the compound is characterized by a binding constant measured by competitive displacement by the compound of omega-conotoxin MVIIA from neuronal membranes.

9. The method of Claim 7, wherein the binding affinity of the compound is characterized by a ratio of binding constants of the compound for the neuronal-cell omega-conotoxin MVIIA site and for neuronal-cell omega-conotoxin SVIB binding site.

## Patentansprüche (Patentansprüche für folgende(n) Vertragsstaat(en): AT, BE, CH, DK, FR, GB, IT, LI, LU, NL, SE)

1. Verwendung eines Antagoristenpeptids des neuronalen Calciumkanals mit Aktivitäten zum Blockieren der Norepinephrin-Freisetzung in neuronalen Zellen des zentralen Nervensystems von Säugern und zum Binden an eine omega-Conotoxin-MVIIA-Bindungsstelle der neuronalen Membran, welche innerhalb des Bereichs solcher Aktivitäten für omega-Conotoxin-MVIIA, -GVIA oder -TVIA liegen, für die Herstellung eines Medikamentes zur Verringerung des mit einem ischämischen Zustand in Säugern in Verbindung stehenden neuronalen Schadens.

2. Verwendung gemäß Anspruch 1, worin das Antagonistenpeptid des neuronalen Calciumkanals ein omega-Conotoxin-Peptid ist.

3. Verwendung gemäß Anspruch 2, worin die Aktivität des Bindens durch das Verhältnis der Bindungskonstanten des Peptids für die omega-Conotoxin-MVIIA-Stelle neuronaler Zellen und für eine omega-Conotoxin-SVIB-Bindungsstelle neuronaler Zellen gekennzeichnet ist, welches innerhalb des Bereichs jener für die omega-Conotoxin-Peptide MVIIA, GVIA oder TVIA bestimmten Verhältnisse liegt.

4. Verwendung gemäß Anspruch 2, worin das Peptid die Form:
CKGKGAX₁CX₂RX₃X₄YDCCTGSCX₅RX₆GKC-t besitzt, worin X₁=K oder S; X₂=S oder H; X₃=L oder T; X₄=M oder S; X₅=N oder eine Deletion; X₆=S oder eine Deletion; und t=Carboxy oder eine amidierte Carboxyendgruppe ist.

5. Verwendung gemäß Anspruch 2, worin das Peptid folgende Form besitzt:
CX₁SXGSSCSXTSYNCCRSCNXYX₂X₃X₄CX₅-t, worin X₁=K oder L; X₂=T oder S; X₃=K oder R; X₄=R oder K; X₅=Y oder R; und t=Carboxy oder eine amidierte Carboxyendgruppe ist.

6. Verwendung gemäß Anspruch 2, worin das Peptid aus der Gruppe gewählt wird, die aus den omega-Conotoxinen MVIIA, GVIA und TVIA besteht.

7. Omega-Conotoxin-Peptid folgender Form:
CKGKGAX₁CX₂RX₃X₄YDCCTGSCX₅RX₆GKC-t, worin X₁=K oder S; X₂=S oder H; X₃=L oder T; X₄=M oder S; X₅=N oder eine Deletion; X₆=S oder eine Deletion; und t=Carboxy oder eine amidierte Carboxyendgruppe ist, ausschließlich der Peptide, in denen X₁=K, X₂=S, X₃=L, X₄=M, X₅=Deletion und X₆=S ist; und in denen X₁=S, X₂=H, X₃=T, X₄=S, X₅=N und X₆=Deletion ist.

8. Conotoxin-Peptid folgender Form:
CX₁SXGSSCSXTSYNCCRSCNXYX₂X₃X₄CX₅-t, worin X₁=K oder L; X₂=T oder S; X₃=K oder R; X₄=R oder K; X₅=Y oder R ist, ausschließlich der Peptide, in denen X₁=K, X₂=T, X₃=K, X₄=R, X₅=Y ist; und in denen X₁=L, X₂=S, X₃=R, X₄=K und X₅=R ist.

9. Verfahren der Auswähl einer Antagonisten-Peptidverbindung des Calciumkanals neuronaler Zellen, welche bei der Herstellung eines Medikamentes zur Verringerung eines mit Ischämie in Verbindung stehenden neuronalen Schadens, wie er durch einen Schlaganfall beim Menschen erzeugt wird, verwendet werden kann, umfassend
Durchführung eines Assays für eine Verbindung bezüglich ihrer Fähigkeit, die Norepinephrin-Freisetzung in neuronalen Zellen des zentralen Nervensystems zu inhibieren, und bezüglich ihrer Affinität zur omega-Conotoxin-MVIIA-Bindungsstelle neuronaler Zellen;
Auswählen der im Assay geprüften Verbindung zur Verwendung bei der Herstellung eines solchen Medikamentes, wenn die Verbindung:
(i) wirksam bei der Inhibierung der Norepinephrin-Freisetzung in neuronalen Zellen des zentralen Nervensystems bei einer Konzentration ist, innerhalb der die omega-Conotoxine MVIIA, GVIA und TVIA wirksam zur Inhibierung einer derartigen Norepinephrin-Freisetzung in neuronalen Zellen sind, und
(ii) eine Bindungsaffinität für eine solche omega-Conotoxin-MVIIA-Bindungsstelle besitzt, welche innerhalb des Bereiches der Bindungsaffinitäten für die Bindungsstelle der omega-Conotoxine MVIIA, GVIA oder TVIA liegt.

10. Verfahren nach Anspruch 9, wobei die Bindungsaffinität der Verbindung durch eine Bindungskonstante gekennzeichnet ist, die durch die kompetitive Verdrängung durch die Verbindung des omega-Conotoxins MVIIA von neuronalen Membranen gemessen wird.

11. Verfahren nach Anspruch 9, wobei die Bindungsaffinität der Verbindung durch ein Verhältnis der Bindungskonstanten der Verbindung für die omega-Conotoxin-MVIIA-Stelle neuronaler Zellen und für die omega-Conotoxin-SVIB-Bindungsstelle neuronaler Zellen gekennzeichnet ist.

## Patentansprüche (Patentansprüche für folgende(n) Vertragsstaat(en): DE)

1. Verwendung eines Antagonistenpeptids des neuronalen Calciumkanals mit Aktivitäten zum Blockieren der Norepinephrin-Freisetzung in neuronalen Zellen des zentralen Nervensystems von Säugern und zum Binden an eine omega-Conotoxin-MVIIA-Bindungsstelle der neuronalen Membran, welche innerhalb des Bereichs solcher Aktivitäten für omega-Conotoxin-MVIIA, -GVIA oder -TVIA liegen, für die Herstellung eines Medikamentes zur Behandlung cerebraler Ischämie.

2. Verwendung gemäß Anspruch 1, worin das Antagonistenpeptid des neuronalen Calciumkanals ein omega-Conotoxin-Peptid ist.

3. Verwendung gemäß Anspruch 2, worin die Aktivität des Bindens durch das Verhältnis der Bindungskonstanten des Peptids für die omega-Conotoxin-MVIIA-Stelle neuronaler Zellen und für eine omega-Conotoxin-SVIB-Bindungsstelle neuronaler Zellen gekennzeichnet ist, welches innerhalb des Bereichs jener für die omega-Conotoxin-Peptide MVIIA, GVIA oder TVIA bestimmten Verhältnisse liegt.

4. Verwendung gemäß Anspruch 2, worin das Peptid die Form:
CKGKGAX₁CX₂RX₃X₄YDCCTGSCX₅RX₆GKC-t besitzt, worin X₁=K oder S; X₂=S oder H; X₃=L oder T; X₄=M oder S; X₅=N oder eine Deletion; X₆=S oder eine Deletion; und t=Carboxy oder eine amidierte Carboxyendgruppe ist.

5. Verwendung gemäß Anspruch 2, worin das Peptid folgende Form besitzt:
CX₁SXGSSCSXTSYNCCRSCNXYX₂X₃X₄CX₅-t, worin X₁=K oder L; X₂=T oder S; X₃=K oder R; X₄=R oder K; X₅=Y oder R; und t=Carboxy oder eine amidierte Carboxyendgruppe ist.

6. Verwendung gemäß Anspruch 2, worin das Peptid aus der Gruppe gewählt wird, die aus den omega-Conotoxinen MVIIA, GVIA und TVIA besteht.

7. Omega-Conotoxin-Peptid folgender Form:
CKGKGAX₁CX₂RX₃X₄YDCCTGSCX₅RX₆GKC-t, worin X₁=K oder S; X₂=S oder H; X₃=L oder T; X₄=M oder S; X₅=N oder eine Deletion; X₆=S oder eine Deletion; und t=Carboxy oder eine amidierte Carboxyendgruppe ist, ausschließlich der Peptide, in denen X₁=K, X₂=S, X₃=L, X₄=M, X₅=Deletion und X₆=S ist; und in denen X₁=S, X₂=H, X₃=T, X₄=S, X₅=N und X₆=Deletion ist.

8. Conotoxin-Peptid folgender Form:
CX₁SXGSSCSXTSYNCCRSCNXYX₂X₃X₄CX₅-t, worin X₁=K oder L; X₂=T oder S; X₃=K oder R; X₄=R oder K; X₅=Y oder R ist, ausschließlich der Peptide, in denen X₁=K, X₂=T, X₃=K, X₄=R, X₅=Y ist; und in denen X₁=L, X₂=S, X₃=R, X₄=K und X₅=R ist.

9. Verfahren der Auswähl einer Antagonisten-Peptidverbindung des Calciumkanals neuronaler Zellen, welche bei der Herstellung eines Medikamentes zur Verringerung eines mit Ischämie in Verbindung stehenden neuronalen Schadens, wie er durch einen Schlaganfall beim Menschen erzeugt wird, verwendet werden kann, umfassend
Durchführung eines Assays für eine Verbindung bezuglich ihrer Fähigkeit, die Norepinephrin-Freisetzung in neuronalen Zellen des zentralen Nervensystems zu inhibieren, und bezüglich ihrer Affinität zur omega-Conotoxin-MVIIA-Bindungsstelle neuronaler Zellen;
Auswählen der im Assay geprüften Verbindung zur Verwendung bei der Herstellung eines solchen Medikamentes, wenn die Verbindung:
(i) wirksam bei der Inhibierung der Norepinephrin-Freisetzung in neuronalen Zellen des zentralen Nervensystems bei einer Konzentration ist, innerhalb der die omega-Conotoxine MVIIA, GVIA und TVIA wirksam zur Inhibierung einer derartigen Norepinephrin-Freisetzung in neuronalen Zellen sind, und
(ii) eine Bindungsaffinität für eine solche omega-Conotoxin-MVIIA-Bindungsstelle besitzt, welche innerhalb des Bereiches der Bindungsaffinitäten für die Bindungsstelle der omega-Conotoxine MVIIA, GVIA oder TVIA liegt.

10. Verfahren nach Anspruch 9, wobei die Bindungsaffinität der Verbindung durch eine Bindungskonstante gekennzeichnet ist, die durch die kompetitive Verdrängung durch die Verbindung des omega-Conotoxins MVIIA von neuronalen Membranen gemessen wird.

11. Verfahren nach Anspruch 9, wobei die Bindungsaffinität der Verbindung durch ein Verhältnis der Bindungskonstanten der Verbindung für die omega-Conotoxin-MVIIA-Stelle neuronaler Zellen und für die omega-Conotoxin-SVIB-Bindungsstelle neuronaler Zellen gekennzeichnet ist.

## Patentansprüche (Patentansprüche für folgende(n) Vertragsstaat(en): ES, GR)

1. Verwendung eines Antagonistenpeptids des neuronalen Calciumkanals mit Aktivitäten zum Blockieren der Norepinephrin-Freisetzung in neuronalen Zellen des zentralen Nervensystems von Säugern und zum Binden an eine omega-Conotoxin-MVIIA-Bindungsstelle der neuronalen Membran, welche innerhalb des Bereichs solcher Aktivitäten für omega-Conotoxin-MVIIA, -GVIA oder -TVIA liegen, für die Herstellung eines Medikamentes zur Verringerung des mit einem ischämischen Zustand in Säugern in Verbindung stehenden neuronalen Schadens.

2. Verwendung gemäß Anspruch 1, worin das Antagonistenpeptid des neuronalen Calciumkanals ein omega-Conotoxin-Peptid ist.

3. Verwendung gemäß Anspruch 2, worin die Aktivität des Bindens durch das Verhältnis der Bindungskonstanten des Peptids für die omega-Conotoxin-MVIIA-Stelle neuronaler Zellen und für eine omega-Conotoxin-SVIB-Bindungsstelle neuronaler Zellen gekennzeichnet ist, welches innerhalb des Bereichs jener für die omega-Conotoxin-Peptide MVIIA, GVIA oder TVIA bestimmten Verhältnisse liegt.

4. Verwendung gemäß Anspruch 2, worin das Peptid die Form:
CKGKGAX₁CX₂RX₃X₄YDCCTGSCX₅RX₆GKC-t besitzt, worin X₁=K oder S; X₂=S oder H; X₃=L oder T; X₄=M oder S; X₅=N oder eine Deletion; X₆=S oder eine Deletion; und t=Carboxy oder eine amidierte Carboxyendgruppe ist.

5. Verwendung gemäß Anspruch 2, worin das Peptid folgende Form besitzt:
CX₁SXGSSCSXTSYNCCRSCNXYX₂X₃X₄CX₅-t, worin X₁=K oder L; X₂=T oder S; X₃=K oder R; X₄=R oder K; X₅=Y oder R; und t=Carboxy oder eine amidierte Carboxyendgruppe ist.

6. Verwendung gemäß Anspruch 2, worin das Peptid aus der Gruppe gewählt wird, die aus den omega-Conotoxinen MVIIA, GVIA und TVIA besteht.

7. Verfahren der Auswähl einer Antagonisten-Peptidverbindung des Calciumkanals neuronaler Zellen, welche bei der Herstellung eines Medikamentes zur Verringerung eines mit Ischämie in Verbindung stehenden neuronalen Schadens, wie er durch einen Schlaganfall beim Menschen erzeugt wird, verwendet werden kann, umfassend
Durchführung eines Assays für eine Verbindung bezuglich ihrer Fähigkeit, die Norepinephrin-Freisetzung in neuronalen Zellen des zentralen Nervensystems zu inhibieren, und bezüglich ihrer Affinität zur omega-Conotoxin-MVIIA-Bindungsstelle neuronaler Zellen;
Auswählen der im Assay geprüften Verbindung zur Verwendung bei der Herstellung eines solchen Medikamentes, wenn die Verbindung:
(i) wirksam bei der Inhibierung der Norepinephrin-Freisetzung in neuronalen Zellen des zentralen Nervensystems bei einer Konzentration ist, innerhalb der die omega-Conotoxine MVIIA, GVIA und TVIA wirksam zur Inhibierung einer derartigen Norepinephrin-Freisetzung in neuronalen Zellen sind, und
(ii) eine Bindungsaffinität für eine solche omega-Conotoxin-MVIIA-Bindungsstelle besitzt, welche innerhalb des Bereiches der Bindungsaffinitäten für die Bindungsstelle der omega-Conotoxine MVIIA, GVIA oder TVIA liegt.

8. Verfahren nach Anspruch 7, wobei die Bindungsaffinität der Verbindung durch eine Bindungskonstante gekennzeichnet ist, die durch die kompetitive Verdrängung durch die Verbindung des omega-Conotoxins MVIIA von neuronalen Membranen gemessen wird.

9. Verfahren nach Anspruch 7, wobei die Bindungsaffinität der Verbindung durch ein Verhältnis der Bindungskonstanten der Verbindung für die omega-Conotoxin-MVIIA-Stelle neuronaler Zellen und für die omega-Conotoxin-SVIB-Bindungsstelle neuronaler Zellen gekennzeichnet ist.

## Revendications (Revendications pour l'(les) Etat(s) contractant(s) suivant(s): AT, BE, CH, DK, FR, GB, IT, LI, LU, NL, SE)

1. Utilisation d'un peptide antagoniste de canal calcique neuronal ayant des activités de blocage de la libération de norépinéphrine dans des cellules neuronales du système nerveux central de mammifère, et de liaison au site de liaison de l'Ω-conotoxine MVIIA de membrane neuronale, qui sont comprises dans la gamme de telles activités pour l'Ω-conotoxine MVIIA, GVIA, ou TVIA, pour la préparation d'un médicament destiné à réduire les dommages neuronaux liés à un trouble ischémique chez une espèce de mammifère.

2. Utilisation selon la revendication 1, dans laquelle le peptide antagoniste de canal calcique neuronal est un peptide d'Ω-conotoxine.

3. Utilisation selon la revendication 2, dans laquelle l'activité de liaison est caractérisée par un rapport de constantes de liaison du peptide pour le site de l'Ω-conotoxine MVIIA de cellule neuronale et pour un site de liaison de l'Ω-conotoxine SVIB de cellule neuronale qui est dans la gamme de tels rapports mesurés pour les peptides d'Ω-conotoxine MVIIA, GVIA, ou TVIA.

4. Utilisation selon la revendication 2, dans laquelle le peptide a la forme :
CKGKGAX₁CX₂RX₃X₄YDCCTGSCX₅RX₆GKC-t, où X₁=K ou S ; X₂=S ou H ; X₃=L ou T ; X₄=M ou S ; X₅=N ou une délétion; X6=S ou une délétion ; et t=un groupe carboxy ou carboxy amidé terminal.

5. Utilisation selon la revendication 2, dans laquelle le peptide a la forme :
CX₁SXGSSCSXTSYNCCRSCNXYX₂X₃X₄CX₅-t, où X₁=K où L ; X₂=T ou S ; X₃=K ou R ; X₄=R ou K ; X₅=Y ou R ; et t=un groupe carboxy ou carboxy amidé terminal.

6. Utilisation selon la revendication 2, dans laquelle le peptide est choisi dans le groupe constitué par des Ω-conotoxines MVIIA, GVIA et TVIA.

7. Peptide d'Ω-conotoxine ayant la forme :
CKGKGAX₁CX₂RX₃X₄YDCCTGSCX₅RX₆GKC-t, où X₁=K ou S ; X₂=S ou H ; X₃=L ou T ; X₄=M ou S ; X₅=N ou une délétion ; X₆=S ou une délétion ; et t=un groupe carboxy ou carboxy amidé terminal, à l'exclusion des peptides dans lesquels X₁=K, X₂=S, X₃=L, X₄=M, X₅=une délétion, et X₆=S ; et X₁=S, X₂=H, X₃=T, X₄=S, X₅=N, et X₆=une délétion.

8. Peptide de conotoxine ayant la forme :
CX₁SXGSSCSXTSYNCCRSCNXYX₂X₃X₄CX₅-t, où X₁=K ou L ; X₂=T ou S ; X₃=K ou R ; X₄=R ou K ; X₅=Y ou R ; à l'exclusion des peptides dans lesquels X₁=K, X₂=T, X₃=K, X₄=R, X₅=Y ; et X₁=L, X₂=S, X₃=R, X₄=K, et X₅=R.

9. Méthode de sélection d'un composé peptidique antagoniste de canal calcique de cellule neuronale qui peut être utilisé dans la préparation d'un médicament pour réduire les dommages neuronaux liés à l'ischémie, tels que produits par une attaque, chez l'homme, comprenant les étapes consistant à
tester la capacité d'un composé à inhiber la libération de norépinéphrine dans des cellules neuronales du système nerveux central, et son affinité pour le site de liaison de l'Ω-conotoxine MVIIA de cellule neuronale,
sélectionner le composé testé pour une utilisation dans la préparation d'un tel médicament si le composé :
(i) inhibe efficacement la libération de norépinéphrine dans des cellules neuronales du système nerveux central, à une concentration dans laquelle les Ω-conotoxines MVIIA, GVIA et TVIA inhibent efficacement une telle libération de norépinéphrine dans des cellules neuronales, et
(ii) a une affinité de liaison pour un tel site de liaison de l'Ω-conotoxine MVIIA qui est comprise dans la gamme des affinités de liaison pour le site de liaison des Ω-conotoxines MVIIA, GVIA, ou TVIA.

10. Méthode de la revendication 9, dans laquelle l'affinité de liaison du composé est caractérisée par une constante de liaison mesurée par déplacement compétitif par le composé d'Ω-conotoxine MVIIA de membranes neuronales.

11. Méthode de la revendication 9, dans laquelle l'affinité de liaison du composé est caractérisée par un rapport de constantes de liaison du composé pour le site de l'Ω-conotoxine MVIIA de cellule neuronale et pour le site de liaison de l'Ω-conotoxine SVIB de cellule neuronale.

## Revendications (Revendications pour l'(les) Etat(s) contractant(s) suivant(s): DE)

1. Utilisation d'un peptide antagoniste de canal calcique neuronal ayant des activités de blocage de la libération de norépinéphrine dans des cellules neuronales du système nerveux central de mammifère, et de liaison au site de liaison de l'Ω-conotoxine MVIIA de membrane neuronale, qui sont comprises dans la gamme de telles activités pour l'Ω-conotoxine MVIIA, GVIA ou TVIA, pour la préparation d'un médicament pour le traitement de l'ischémie cérébrale.

2. Utilisation selon la revendication 1, dans laquelle le peptide antagoniste de canal calcique neuronal est un peptide d'Ω-conotoxine.

3. Utilisation selon la revendication 2, dans laquelle l'activité de liaison est caractérisée par un rapport de constantes de liaison du peptide pour le site de l'Ω-conotoxine MVIIA de cellule neuronale et pour un site de liaison de l'Ω-conotoxine SVIB de cellule neuronale qui est dans la gamme de tels rapports mesurés pour les peptides d'Ω-conotoxine MVIIA, GVIA, ou TVIA.

4. Utilisation selon la revendication 2, dans laquelle le peptide a la forme :
CKGKGAX₁CX₂RX₃X₄YDCCTGSCX₅RX₆GKC-t, où X₁=K ou S ; X₂=S ou H ; X₃=L ou T ; X₄=M ou S ; X₅=N ou une délétion ; X₆=S ou une délétion ; et t=un groupe carboxy ou carboxy amidé terminal.

5. Utilisation selon la revendication 2, dans laquelle le peptide a la forme :
CX₁SXGSSCSXTSYNCCRSCNXYX₂X₃X₄CX₅-t, où X₁=K ou L ; X₂=T ou S ; X₃=K ou R ; X₄=R ou K ; X₅=Y ou R ; et t=un groupe carboxy ou carboxy amidé terminal.

6. Utilisation selon la revendication 2, dans laquelle le peptide est choisi dans le groupe constitué par des Ω-conotoxines MVIIA, GVIA, et TVIA.

7. Peptide d'Ω-conotoxine ayant la forme :
CKGKGAX₁CX₂RX₃X₄YDCCTGSCX₅RX₆GKC-t, où X₁=K ou S ; X₂=S ou H ; X₃=L ou T ; X₄=M ou S ; X₅=N ou une délétion ; X₆=S ou une délétion; et t=un groupe carboxy ou carboxy amidé terminal, à l'exclusion des peptides dans lesquels X₁=K, X₂=S, X₃=L, X₄=M, X₅=une délétion, et X₆=S ; et X₁=S, X₂=H, X₃=T, X₄=S, X₅=N, et X₆=une délétion.

8. Peptide de conotoxine ayant la forme :
CX₁SXGSSCSXTSYNCCRSCNXYX₂X₃X₄CX₅-t, où X₁=K ou L ; X₂=T ou S ; X₃=K ou R ; X₄=R ou K ; X₅=Y ou R ; à l'exclusion des peptides dans lesquels X₁=K, X₂=T, X₃=K, X₄=R, X₅=Y ; et X₁=L, X₂=S, X₃=R, X₄=K, et X₅=R.

9. Méthode de sélection d'un composé peptidique antagoniste de canal calcique de cellule neuronale qui peut être utilisé dans la préparation d'un médicament destiné à réduire les dommages neuronaux liés à l'ischémie, tels que produits par une attaque, chez l'homme, comprenant les étapes consistant à
tester la capacité d'un composé à inhiber la libération de norépinéphrine dans des cellules neuronales du système nerveux central et son affinité pour le site de liaison de l'Ω-conotoxine MVIIA de cellule neuronale,
sélectionner le composé testé pour une utilisation dans la préparation d'un tel médicament si le composé :
(i) inhibe efficacement la libération de norépinéphrine dans des cellules neuronales du système nerveux central, à une concentration dans laquelle les Ω-conotoxines MVIIA, GVIA, et TVIA inhibent efficacement une telle libération de norépinéphrine dans des cellules neuronales, et
(ii) a une affinité de liaison pour un tel site de liaison de l'Ω-conotoxine MVIIA qui est comprise dans la gamme des affinités de liaison pour le site de liaison des Ω-conotoxines MVIIA, GVIA ou TVIA.

10. Méthode de la revendication 9, dans laquelle l'affinité de liaison du composé est caractérisée par une constante de liaison mesurée par déplacement compétitif par le composé d'Ω-conotoxine MVIIA de membranes neuronales.

11. Méthode de la revendication 9, dans laquelle l'affinité de liaison du composé est caractérisée par un rapport de constantes de liaison du composé pour le site de l'Ω-conotoxine MVIIA de cellule neuronale et pour le site de liaison de l'Ω-conotoxine SVIB de cellule neuronale.

## Revendications (Revendications pour l'(les) Etat(s) contractant(s) suivant(s): ES, GR)

1. Utilisation d'un peptide antagoniste de canal calcique neuronal ayant des activités de blocage de la libération de norépinéphrine dans des cellules neuronales du système nerveux central de mammifère, et de liaison au site de liaison de l'Ω-conotoxine MVIIA de membrane neuronale, qui sont comprises dans la gamme de telles activités pour l'Ω-conotoxine MVIIA, GVIA, ou TVIA, pour la préparation d'un médicament destiné à réduire les dommages neuronaux liés à un trouble ischémique chez une espèce de mammifère.

2. Utilisation selon la revendication 1, dans laquelle le peptide antagoniste de canal calcique neuronal est un peptide d'Ω-conotoxine.

3. Utilisation selon la revendication 2, dans laquelle l'activité de liaison est caractérisée par un rapport de constantes de liaison du peptide pour le site de l'Ω-conotoxine MVIIA de cellule neuronale et pour un site de liaison de l'Ω-conotoxine SVIB de cellule neuronale qui est dans la gamme de tels rapports mesurés pour les peptides d'Ω-conotoxine MVIIA, GVIA, ou TVIA.

4. Utilisation selon la revendication 2, dans laquelle le peptide a la forme :
CKGKGAX₁CX₂RX₃X₄YDCCTGSCX₅RX₆GKC-t, où X₁=K ou S ; X₂=S ou H ; X₃=L ou T ; X₄=M ou S ; X₅=N ou une délétion ; X₆=S ou une délétion; et t=un groupe carboxy ou carboxy amidé terminal.

5. Utilisation selon la revendication 2, dans laquelle le peptide a la forme :
CX₁SXGSSCSXTSYNCCRSCNXYX₂X₃X₄CX₅-t, où X₁=K ou L ; X₂=T ou S ; X₃=K ou R ; X₄=R ou K ; X₅=Y ou R ; et t=un groupe carboxy ou carboxy amidé terminal.

6. Utilisation selon la revendication 2, dans laquelle le peptide est choisi dans le groupe constitué par des Ω-conotoxines MVIIA, GVIA, et TVIA.

7. Méthode de sélection d'un composé peptidique antagoniste de canal calcique de cellule neuronale qui peut être utilisé dans la préparation d'un médicament destiné à réduire les dommages neuronaux liés à l'ischémie, tels que produits par une attaque, chez l'homme, comprenant les étapes consistant à
tester la capacité d'un composé à inhiber la libération de norépinéphrine dans des cellules neuronales du système nerveux central, et son affinité pour le site de liaison de l'Ω-conotoxine MVIIA de cellule neuronale,
sélectionner le composé testé pour une utilisation dans la préparation d'un tel médicament si le composé :
(i) inhibe efficacement la libération de norépinéphrine dans des cellules neuronales du système nerveux central, à une concentration dans laquelle les Ω-conotoxines MVIIA, GVIA, et TVIA inhibent efficacement une telle libération de norépinéphrine dans des cellules neuronales, et
(ii) a une affinité de liaison pour un tel site de liaison de l'Ω-conotoxine MVIIA qui est comprise dans la gamme des affinités de liaison pour le site de liaison des Ω-conotoxines MVIIA, GVIA, ou TVIA.

8. Méthode de la revendication 7, dans laquelle l'affinité de liaison du composé est caractérisée par une constante de liaison mesurée par déplacement compétitif par le composé d'Ω-conotoxine MVIIA de membranes neuronales.

9. Méthode de la revendication 7, dans laquelle l'affinité de liaison du composé est caractérisée par un rapport de constantes de liaison du composé pour le site de l'Ω-conotoxine MVIIA de cellule neuronale et pour le site de liaison de l'Ω-conotoxine SVIB de cellule neuronale.
